(19) Europäisches Patentamt
European Patent Office
Office européen des brevets

(11) Veröffentlichungsnummer : **0 387 195 B1**

# (12) EUROPÄISCHE PATENTSCHRIFT

(45) Veröffentlichungstag der Patentschrift :
**27.07.94 Patentblatt 94/30**

(51) Int. Cl.$^5$ : **C07D 285/14,** C07D 417/12, A01N 43/82

(21) Anmeldenummer : **90810151.2**

(22) Anmeldetag : **27.02.90**

## (54) Mittel zum Schutz von Pflanzen gegen Krankheiten.

(30) Priorität : **08.03.89 CH 864/89**

(43) Veröffentlichungstag der Anmeldung :
**12.09.90 Patentblatt 90/37**

(45) Bekanntmachung des Hinweises auf die Patenterteilung :
**27.07.94 Patentblatt 94/30**

(84) Benannte Vertragsstaaten :
**AT BE CH DE DK ES FR GB GR IT LI LU NL SE**

(56) Entgegenhaltungen :
**EP-A- 0 061 836**
**EP-A- 0 174 088**
**EP-A- 0 246 507**
**EP-A- 0 268 892**

(56) Entgegenhaltungen :
**EP-A- 0 270 971**
**EP-A- 0 292 937**
**DE-A- 1 695 786**
**GB-A- 1 177 972**

(73) Patentinhaber : **CIBA-GEIGY AG**
**Klybeckstrasse 141**
**CH-4002 Basel (CH)**

(72) Erfinder : **Kunz, Walter, Dr.**
**Buchenstrasse 9**
**CH-4104 Oberwil (CH)**
Erfinder : **Schurter, Rolf, Dr.**
**Holzmattstrasse 45**
**CH-4102 Binningen (CH)**
Erfinder : **Nyfeler, Robert, Dr.**
**Bärenfelserstrasse 8**
**CH-4057 Basel (CH)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue N-(Cyanomethyl)-benz-1,2,3-thiadiazol-7-carbonsäureamide der nachstehenden Formel I. Die Erfindung betrifft ferner die Herstellung dieser Substanzen sowie die als Wirkstoff mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

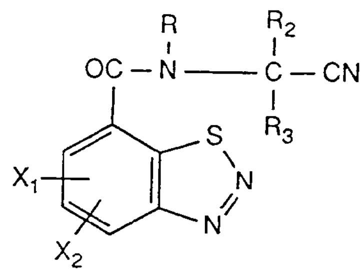

(I)

in welcher bedeuten:

$X_1$ und $X_2$ unabhängig voneinander Wasserstoff oder Halogen;
R Wasserstoff, $C_1$-$C_4$-Alkyl, $C_3$-$C_5$-Alkenyl oder $SR_1$;
$R_1$ mit 3 bis 6 Fluor oder Chlor substituiertes $C_1$-$C_3$-Alkyl;
$R_2$ $C_1$-$C_4$-Alkyl, durch Sauerstoff unterbrochenes $C_1$-$C_5$-Alkyl, $C_1$-$C_6$-Alkoxy, mit 1 bis 3 Halogen substituiertes $C_1$-$C_3$-Alkyl oder substituiertes $C_2$-$C_3$-Alkoxy, $C_1$-$C_6$-Alkylthio, in 2- oder 3-Stellung gebundenes Furyl oder gebundenes Thienyl, mit 1 bis 3 Halogen substituiertes und in 2- oder 3-Stellung gebundenes Furyl oder gebundenes Thienyl; und
$R_3$ Wasserstoff oder $C_1$-$C_4$-Alkyl.

Halogen bedeutet Fluor, Chlor, Brom oder Jod, bevorzugt Fluor und weiter in der Reihenfolge Chlor, Brom und Jod.

Unter Alkyl selbst oder als Bestandteil eines anderen Substituenten sind geradkettige und verzweigte Alkyle zu verstehen. Sie stellen je nach Zahl der angegebenen Kohlenstoffatome beispielsweise folgende Gruppen dar: Methyl, Ethyl sowie die Isomeren von Propyl oder Butyl, wie z.B. Isopropyl, Isobutyl, tert.-Butyl oder sek.-Butyl.

Alkenyl steht z.B. für Propenyl-(1), Allyl, Butenyl-(1), Butenyl-(2) oder Butenyl-(3).

Die Erfindung bezieht sich insbesondere auf Verbindungen der Formel I, in welcher bedeuten:

$X_1$ und $X_2$ unabhängig voneinander Wasserstoff oder Fluor;
R Wasserstoff, Methyl, Ethyl, Allyl oder den Rest S-$R_1$;
$R_1$ Trichlormethyl, Dichlorfluormethyl oder 1,1,2,2-Tetrachlorethyl.

Ferner sind Verbindungen der Formel I bevorzugt, in welcher bedeuten:

$X_1$ und $X_2$ unabhängig voneinander Wasserstoff oder Fluor;
R Wasserstoff;
$R_2$ $C_1$-$C_4$-Alkoxy, $C_1$-$C_4$-Alkylthio, in 2- oder 3-Stellung gebundenes Furyl oder Thienyl, mit 1 bis 3 Halogen substituiertes und in 2- oder 3-Stellung gebundenes Furyl oder gebundenes Thienyl; und
$R_3$ Wasserstoff oder $C_1$-$C_3$-Alkyl.

Aufgrund ihrer besonderen pflanzenschützenden Eigenschaften lassen sich die Verbindungen der Formel I in folgende Gruppen einteilen:

1. Verbindungen der Formel I, worin bedeuten:

$X_1$ und $X_2$ unabhängig voneinander Wasserstoff oder Fluor;
R Wasserstoff, Methyl, Ethyl, Allyl oder den Rest S-$R_1$;
$R_1$ Trichlormethyl, Dichlorfluormethyl oder 1,1,2,2-Tetrachlorethyl;
$R_2$ $C_1$-$C_4$-Alkyl, durch Sauerstoff unterbrochenes $C_1$-$C_5$-Alkyl, $C_1$-$C_6$-Alkoxy, Trichlormethyl, Trifluormethyl, 1,1,1-Trifluorethoxy, $C_1$-$C_6$-Alkylthio, in 2- oder 3-Stellung gebundenes Furyl oder gebundenes Thienyl, mit 1 bis 3 Halogen substituiertes und in 2- oder 3-Stellung gebundenes Furyl oder gebundenes Thienyl; und

$R_3$ Wasserstoff oder methyl.

2. Verbindungen der Formel I, worin bedeuten:

$X_1$ und $X_2$ unabhängig voneinander Wasserstoff oder Fluor;

R Wasserstoff, Methyl, Ethyl, Allyl oder den Rest S-$R_1$;

$R_1$ Trichlormethyl, Dichlorfluormethyl oder 1,1,2,2-Tetrachlorethyl;

$R_2$ $C_1$-$C_4$-Alkyl, durch Sauerstoff unterbrochenes $C_1$-$C_5$-Alkyl, $C_1$-$C_6$-Alkoxy, Trichlormethyl, Trifluormethyl, 1,1,1-Trifluorethoxy, $C_1$-$C_6$-Alkylthio, in 2- oder 3-Stellung gebundenes Furyl oder Thienyl, mit 1 bis 3 Halogen substituiertes und in 2- oder 3-Stellung gebundenes Furyl oder gebundenes Thienyl; und

$R_3$ Wasserstoff.

Verbindungen der Formel I, worin bedeuten:

$X_1$ und $X_2$ unabhängig voneinander Wasserstoff oder Fluor;

R Wasserstoff;

$R_2$ $C_1$-$C_6$-Alkoxy, $C_1$-$C_6$-Alkylthio oder in 2- oder 3-Stellung gebundenes Furyl oder gebundenes Thienyl; und

$R_3$ Wasserstoff.

Die folgenden Verbindungen zeichnen sich durch besonders vorteilhafte pflanzenschützende Eigenschaften aus:

N-(Benz-1,2,3-thiadiazol-7-carbonyl)-2-ethoxy-2-aminoacetonitril;
N-(Benz-1,2,3-thiadiazol-7-carbonyl)-2-methoxy-2-aminoacetonitril;
N-(Benz-1,2,3-thiadiazol-7-carbonyl)-2-(furyl-2′yl)-2-aminoacetonitril;
N-(Benz-1,2,3-thiadiazol-7-carbonyl)-2-(thiophen-2′yl)-2-aminoacetonitril;
N-(Benz-1,2,3-thiadiazol-7-carbonyl)-2-(thiophen-3′yl)-2-aminoacetonitril;
N-(5-Fluor-benz-1,2,3-thiadiazol-7-carbonyl)-2-(thiophen-3′yl)-2-aminoacetonitril;
N-(4-Fluor-benz-1,2,3-thiadiazol-7-carbonyl)-2-(thiophen-3′yl)-2-aminoacetonitril;
N-(Benz-1,2,3-thiadiazol-7-carbonyl)-2-(furyl-3′yl)-2-aminoacetonitril;
N-(Benz-1,2,3-thiadiazol-7-carbonyl)-2-methylthio-2-aminoacetonitril;
N-(Benz-1,2,3-thiadiazol-7-carbonyl)-2-isopropylthio-2-aminoacetonitril.

Heteroaryl substituierte N-(Cyanomethyl)carbonsäureamide mit fungiziden Eigenschaften sind bekannt, z.B. aus EP-A-268892 und EP-A-292937.

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mikroorganismen mittels Blattapplikation oder mittels Bodenapplikation als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I eigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden: Pilze, wie z.B. Oomyceten (z.B. Plasmopara viticola, Phytophthora infestans, Peronospora tabacina, Pseudoperonospora, Bremia letucae), Fungi imperfecti (z.B. Colletotrichum lagenarium, Piricularia oryzae, Cercospora nicotinae), Ascomyceten (z.B. Venturia inaequalis); Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen unterschiedlicher Nutzkulturen eingesetzt werden.

Für den Einsatz der erfindungsgemässen Verbindungen der Formel I im Rahmen der Erfindung sind beispielsweise folgende Pflanzenarten geeignet: Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurken, Tabak, Reben, Reis, Pfeffer, Kartoffeln, Tomaten, Weizen, Gerste, Birnen und Aepfel.

Verbindungen der Formel I mit Ausnahme derjenigen Verbindungen, in denen R für den Rest S-$R_1$ und $R_2$ für $C_1$-$C_6$-Alkoxy oder $C_1$-$C_6$-Alkylthio stehen, werden hergestellt, indem man umsetzt: eine Benzthiadiazolverbindung der Formel II

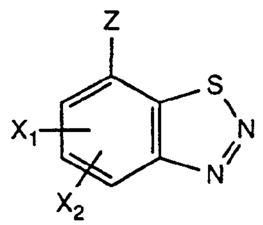

(II)

mit einer Aminoverbindung der Formel III

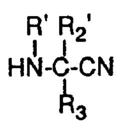

(III)

in Gegenwart einer Base und gegebenenfalls mit einem Katalysator, wie z.B. 4-Dialkylaminopyridin speziell 4-Dimethylaminopyridin, in inerten Lösungsmitteln, wobei Z die Reste COOH, Hal-CO, $COOC_1$-$C_5$-Alkyl,

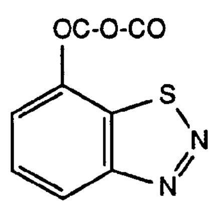

oder

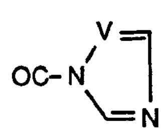

bedeutet, V CH oder N und Hal Halogen darstellen und R′ die Bedeutung von R unter Formel I mit Ausnahme von S-$R_1$ und $R_2$′ die Bedeutung von $R_2$ unter Formel I mit Ausnahme von $C_1$-$C_6$-Alkoxy oder $C_1$-$C_6$-Alkylthio haben und $X_1$, $X_2$ und $R_3$ die unter der Formel I angegebenen Bedeutungen besitzen. Die Reaktion findet bei Temperaturen von -10° bis 160°C, bevorzugt von 0° bis 100°C, statt.

Verbindungen der Formel I, in denen für R die Bedeutung S-$R_1$ ausgeschlossen ist (=R′) und in welchen $R_2$ $C_1$-$C_6$-Alkoxy oder $C_1$-$C_6$-Alkylthio (=$R_2$″) bedeutet, werden hergestellt, indem man: a) eine Benzthiadiazolverbindung der Formel II

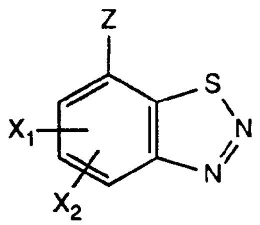

(II)

mit einer Aminoverbindung der Formel III'

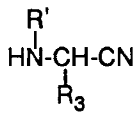

(III')

in Gegenwart einer Base in inerten Lösungsmitteln bei Temperaturen von -10° bis 160°C, bevorzugt von 0° bis 100°C, zu einer Amidverbindung der Formel IV

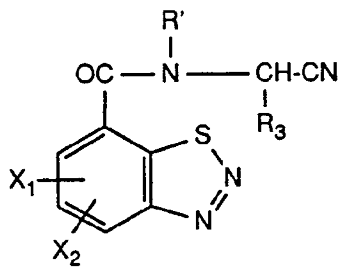

(IV)

umsetzt und anschliessend

b) die Verbindung der Formel IV mit einem Halogenierungsmittel in inerten Lösungsmitteln bei Temperaturen von -30° bis 70°C, bevorzugt von 0° bis 40°C, in eine Halogenverbindung der Formel V

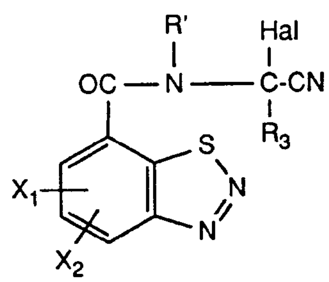

(V)

überführt und dann

c) die Verbindung der Formel V nach Isoliervng oder ohne Isolierung mit einem Alkohol oder Thiol der Formel VI

$HR_2''$ (VI)

in Gegenwart einer Base in inerten Lösungsmitteln bei Temperaturen von -20° bis 160°C, bevorzugt von 0° bis 100°C, verethert, wobei Z die Reste COOH, Hal-CO, $COOC_1$-$C_3$-Alkyl,

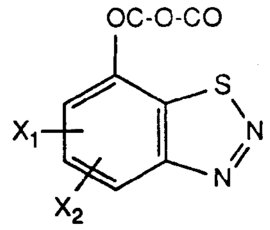 oder 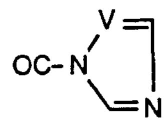

bedeutet, V CH oder N und Hal Halogen darstellen und R′ die Bedeutung von R unter Formel I mit Ausnahme von S-$R_1$ hat und $R_2''$ die Bedeutung von $C_1$-$C_6$-Alkoxy und $C_1$-$C_6$-Alkylthio hat und $X_1$, $X_2$ und $R_3$ die unter Formel I angegebenen Bedeutungen besitzt.

Verbindungen der Formel I, in welcher R die Bedeutung von S-$R_1$ hat, werden synthetisiert, indem man eine Verbindung der Formel Ia

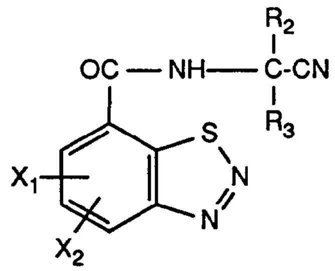

(Ia),.

hergestellt nach dem vorstehenden Verfahren (a), wobei R′ in Formel IV Wasserstoff darstellt, mit einem Sulfenylhalogenid der Formel VII

Hal-S-$R_1$ (VII)

in Gegenwart einer Base in inerten Lösungsmitteln bei Temperaturen von -10° bis 80°C, bevorzugt von 0° bis 50°C, umsetzt, wobei Hal Halogen, insbesondere Chlor, bedeutet und $X_1$, $X_2$, $R_1$, $R_2$ und $R_3$ die unter der Formel I angegebenen Bedeutungen besitzt.

Verbindungen der Formel II, in denen Z COOH bedeutet, können entweder wie in der Literatur beschrieben (vgl. J.Chem.Soc. 1971,3997) oder vorteilhafterweise gemäss den nachstehend angegebenen Herstellungsbeispielen erhalten werden. Säurehalogenide, die unter die Formel II fallen, werden aus den entsprechenden freien Carbonsäuren z.B. mit Thionylchlorid, Phosgen, Oxalylchlorid oder 1-Chlor-N,N-2-trimethylpropenylamin (vgl. L. Ghosez, J.Chem.Soc. Comm. 1979, 1180) hergestellt. Säureanhydride, die unter die Formel II fallen, können z.B. durch Erwärmen der entsprechenden freien Säure mit Acetanhydrid erhalten werden. Imidazolide und Triazolide, die unter die Formel II fallen, werden aus den Carbonsäuren durch Umsetzung mit N,N-Carbonyldiimidazol oder N,N-Carbonylditriazol gewonnen (vgl. H.A. Staab, Angew. Chemie 1964, 132).

Als Basen kommen organische und anorganische in Betracht, z.B. tertiäre Amine wie Trialkylamine (Trimethylamin, Triethylamin, Tripropylamin usw.), Pyridinbasen (Pyridin, 4-Dimethylaminopyridin, 4-Pyrrolidylaminopyridin, Collidin), Hydroxide, Carbonate und Hydrogencarbonate von Alkali- und Erdalkalimetallen sowie Alkaliacetate.

Als Reaktionsmedien in Anpassung an die jeweiligen Reaktionsbedingungen werden geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Dichlormethan, Chloroform, Tetrachlorkohlenstoff, 1,1,1-Trichlorethan, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Ketone wie Aceton, Diethylketon, Methylethylketon; sowie Gemische solcher Lösungsmittel untereinander.

Unter den in den vorgängig beschriebenen Verfahren gebrauchten Halogenierungsmitteln sind z.B. zu verstehen Chlorgas, Sulfurylchlorid, Brom, Trimethylammoniumperbromid und Phosphortribromid.

Aminoverbindungen der Formeln III und III′ sowie Sulfenylhalogenide der Formel VII sind bekannt oder lassen sich nach bekannten Methoden herstellen.

Die im Rahmen der Erfindung zur Anwendung gelangenden mikrobiziden Mittel zum Schutz von Pflanzen

gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation-und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkalirest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I,99,9 bis 1 Gew.-%, insbesondere 99,8 bis 5 Gew.-%, eines festen oder flüssigen Zusatzstofles und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

1. Herstellungsbeispiele

1.1 Herstellung von N-(Benz-1,2,3-thiadiazol-7-carbonyl)-2-ethoxy-aminoacetonitril (Verb. 1.2)

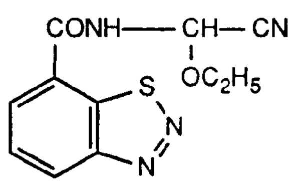

10 g N-Cyanomethyl-benz-1.2.3-thiadiazol-7-carbonsäureamid werden in 140 ml Tetrahydrofuran und 140 ml Essigsäureethylester suspendiert, und bei Raumtemperatur nacheinander mit 5 Tropfen Bromwasserstoffsäure (konzentrierte Lösung in Eisessig) und 2.4 ml Brom versetzt. Dann wird langsam auf 35-40°C erwärmt, wobei die Reaktion einsetzt. Es wird noch 1 Stunde bei 40°C gerührt, anschliessend auf -40°C abgekühlt und bei dieser Temperatur innerhalb 1/2 Stunde tropfenweise mit einer Lösung von 11.4 g Ethanol und 12.0 ml Triethylamin in 10 ml Essigsäureethylester versetzt. Dann wird bis zur vollständigen Umsetzung bei gleichzeitigem Anstieg auf Raumtemperatur gerührt. Zur Aufarbeitung wird von ungelöstem Salz abfiltriert, das Filtrat eingedampft und an Kieselgel (Hexan/Tetrahydrofuran 3:1) gereinigt. Das resultierende Produkt wird aus Dichlormethan/Hexan umkristallisiert und schmilzt ab 142°C unter Zersetzung.

1.2 Herstellung von N-(Benz-1.2.3-thiadiazol-7-carbonyl)-aminoacetonitril (Zwischenprodukt)

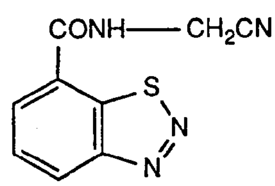

Eine Suspension von 10.2 g Aminoacetonitrilhydrochlorid in 300 ml Dichlormethan wird unter Kühlen und Rühren bei 0-5°C nacheinander tropfenweise mit 31 ml Triethylamin und 100 ml einer 1 molaren Lösung von Benz- 1 .2.3-thiadiazol-7-carbonsäurechlorid versetzt. Ueber Nacht wird bei Raumtemperatur gerührt, mit 200 ml Eiswasser versetzt, der entstandene Niederschlag abfiltriert, mit Wasser gewaschen, in Tetrahydrofuran/Hexan 1:1 gelöst und über eine Kieselgelsäule filtriert. Das Filtrat wird eingedampft und der Rückstand aus Isopropanol/Hexan umkristallisiert. Das Produkt hat einen Smp. von 237-242°C.

1.3 Herstellung des symmetrischen Anhydrids der 1,2,3-Benzthiadiazol-7-carbonsäure (Vorstufe)

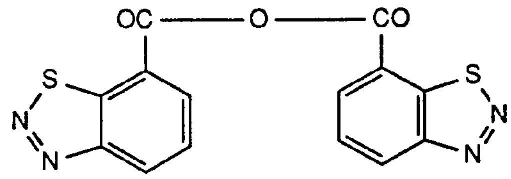

3 g 1,2,3-Benzthiadiazol-7-carbonsäure werden in 50 ml Acetanhydrid während 24 Stunden unter Rückfluss gekocht. Dann wird die dünne Suspension unter Vakuum eingedampft, der feste Rückstand mit Ether aufgeschlämmt und abfiltriert. Es resultieren 4,3 g Anhydrid vom Smp. 117-119°C. Die gleiche Verbindung wird z.B. auch durch Erwärmen der Carbonsäure mit Bis-(2-oxo-3-oxazolidinyl)-phosphin-säurechlorid in trockenem Tetrahydrofuran erhalten (vgl. Synthesis 1981, 616).

1.4 Herstellung von 7-Methoxycarbonyl-benz-1,2,3-thiadiazol (Vorstufe)

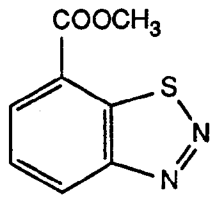

a) 100 g (0,35 Mol) 2-Benzylthio-3,5-diaminobenzoesäuremethylester werden portionsweise zu 250 ml konz. Salzsäure und 110 ml Wasser gegeben und 1,5 Stunden bei Raumtemperatur gerührt. Dann wird das Gemisch auf -5°C gekühlt und während 2,5 Stunden unter Rühren eine Lösung von 48,5 g (0,70 Mol) Natriumnitrit in 210 ml Wasser zugetropft. Der Rührvorgang wird weitere 2 Stunden bei 0°C fortgesetzt. Anschliessend werden 190 ml 50 %ige unterphosphorige Säure während 2 1/2 Stunden zugetropft. Darauf lässt man die Temperatur während 19 Stunden auf 20°C steigen. Das erhaltene Produkt wird abfiltriert, mit Wasser gewaschen und getrocknet.

Zur Reinigung wird das Produkt in Essigsäureethylester/Methylenchlorid gelöst, durch Kieselgel filtriert, evaporiert und durch Zugabe von Hexan kristallisiert. Ausbeute: 44,4 g (65 % d.Th.); Smp. 132°C.

b) 576 g (2 Mol) 3,5-Diamino-2-benzylthio-benzoesäure-methylester werden in 500 ml 1,4-Dioxan gelöst und unter Rühren und Kühlen bei 0° bis 5°C zu vorgelegter 5N Salzsäure (3 l) getropft. Anschliessend wird die feine Suspension auf -17° bis -20°C abgekühlt und innerhalb 1,25 Stunden unter Niveau tropfenweise mit 294 g Natriumnitrit in 500 ml Wasser versetzt. Unter weiterem Rühren wird die Innentemperatur innerhalb 1 Stunde auf -5°C ansteigen gelassen und während 2 Stunden gehalten. Dann wird auf -15°C abgekühlt und die Suspension unter Rühren portionenweise in auf -10° bis -15°C gekühlte Unterphosphorige Säure (1,11) eingetragen, wobei Stickstoff entweicht. Nach beendeter Zugabe wird die Innentemperatur innerhalb 5-6 Stunden auf Raumtemperatur ansteigen gelassen, der gebildete Niederschlag abfiltriert, diese mit 2,5 l Methylenchlorid verrührt, wieder vom nichtgelösten Teil abfiltriert und das Filtrat vom Wasser getrennt. Anschliessend wird die organische Phase über Natriumsulfat getrocknet, mit 300 g Kieselgel verrührt, erneut filtriert, mit Methylenchlorid nachgewaschen und das Filtrat eingedampft. Aus Methanol umkristallisiert resultieren insgesamt 244,8 g (63,1 % d. Th.) beige Kristalle vom Smp. 130°-133°C.

1.5 Herstellung von N-(Trichlormethylthio)-N-(benz-1,2,3-thiadiazol-7-carbonyl)-2-thiophen-3'yl)-2-aminoacetonitril

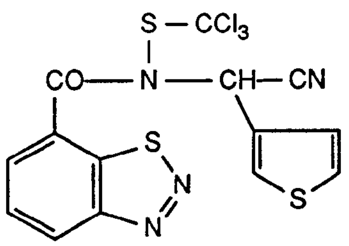

Zur vorgelegten Suspension von 0,8 g N-(Benz-1,2,3-thiadiazol-7-carbonyl)-2-thiophen-3′yl)-2-aminoacetonitril und 0,31 ml Perchlormethylmercaptan in 14 ml Methylenchlorid werden unter Kühlen und Rühren bei 10 bis 15°C eine Lösung von 0,43 ml Triethylamin in 3 ml Methylenchlorid zugetropft. Die entstehende Lösung wird nach Rühren über Nacht mit Eiswasser versetzt und die wässrige Phase mit Methylenchlorid extrahiert. Die Extrakte werden mit Wasser gewaschen und über Natriumsulfat getrocknet. Nach Filtrieren und Eindampfen verbleibt ein öliger Rückstand, der an Kieselgel (Essigsäureethylester/Hexan 1:2) chromatographiert wird. Es resultiert ein Feststoff vom Schmelzpunkt 162-164°C.

Tabelle 1:

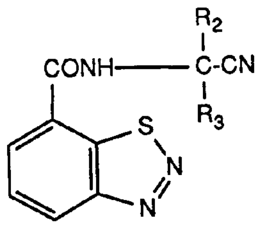

| Verb. Nr. | $R_2$ | $R_3$ | phys. Daten |
|---|---|---|---|
| 1.1 | O-Methyl | H | Smp. 169-172°C (Zers.) |
| 1.2 | O-Ethyl | H | Smp. 142°C (Zers.) |
| 1.3 | O-Propyl-n | H | |
| 1.4 | O-Propyl-i | H | |
| 1.5 | O-Butyl-n | H | |
| 1.6 | O-Butyl-i | H | Smp. 85- 88°C |
| 1.7 | O-Butyl-t | H | |
| 1.8 | O-Pentyl-n | H | |
| 1.9 | O-Pentyl-i | H | |
| 1.10 | O-Hexyl-n | H | Smp. 47- 50°C |
| 1.11 | O-Ethyl | Methyl | |
| 1.12 | 3-Thienyl | H | Smp. 169-172°C |
| 1.13 | O-Ethyl | Ethyl | |
| 1.14 | O-Butyl | Methyl | |
| 1.15 | O-Hexyl-n | Butyl-n | |
| 1.16 | S-Methyl | H | Smp. 142-145°C |
| 1.17 | S-Ethyl | H | |
| 1.18 | S-Ethyl | Methyl | |
| 1.19 | S-Propyl-n | H | |
| 1.20 | S-Propyl-i | H | Smp. 98-101°C |
| 1.21 | S-Hexyl-sec. | Butyl-i | |
| 1.22 | Methyl | H | |
| 1.23 | Ethyl | H | |
| 1.24 | n-Propyl | H | |
| 1.25 | Butyl-i | H | |
| 1.26 | Ethyl | Methyl | |
| 1.27 | Butyl-n | Butyl-n | |
| 1.28 | Methoxymethyl | H | |
| 1.29 | Ethoxyethyl | H | |
| 1.30 | Ethoxymethyl | Methyl | |
| 1.31 | n-Propoxy-ethyl | H | |
| 1.32 | Methoxyethyl | Methyl | |
| 1.33 | Methoxymethyl | Methyl | Smp. 133-135°C |
| 1.34 | 2-Furyl | H | |
| 1.35 | 2-Thienyl | H | |
| 1.36 | 2-Furyl | Methyl | |
| 1.37 | 2-Thienyl | Ethyl | |
| 1.38 | 3-Furyl | H | Smp. 133-135°C |
| 1.39 | 3-Thienyl | Methyl | |
| 1.40 | 3-Furyl | Butyl-n | |

Fortsetzung Tabelle 1:

| Verb. Nr. | $R_2$ | $R_3$ | phys. Daten |
|---|---|---|---|
| 1.41 | 3-Thienyl | Propyl-i | |
| 1.42 | 5'-Chlor-furyl-2 | H | |
| 1.43 | 3',4',5'-Trichlorfuryl-2 | H | |
| 1.44 | 4',5'-Dichlor-furyl-2 | H | |
| 1.45 | 5'-Chlor-furyl-2 | Methyl | |
| 1.46 | 4',5'-Dichlor-furyl-2 | n-Butyl | |
| 1.47 | 2',4'-Dichlor-furyl-3 | H | |
| 1.48 | 2',4',5'-Tribrom-furyl-3 | H | |
| 1.49 | 2',4',5'-Trichlor-furyl-3 | Methyl | |
| 1.50 | 2',5'-Difluor-furyl-3 | H | |
| 1.51 | 5'-Brom-thienyl-2 | H | |
| 1.52 | 3',4',5'-Trichlor-thienyl-2 | H | |
| 1.53 | 3',4',5'-Tribrom-thienyl-2 | n-Propyl | |
| 1.54 | 5'-Chlor-thienyl-3 | H | |
| 1.55 | 2',5'-Dichlor-thienyl-3 | Methyl | |
| 1.56 | 3',4',5'-Tribrom-thienyl-2 | H | |
| 1.57 | 4',5'-Dibrom-furyl-3 | H | |
| 1.58 | 4',5'-Dichlor-furyl-3 | H | |

Tabelle 2:

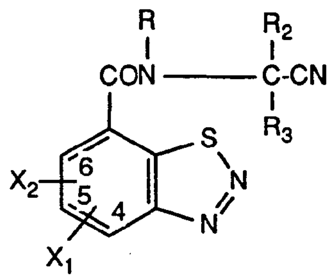

| Verb. Nr. | $X_1$ | $X_2$ | R | $R_2$ | $R_3$ | phys. Daten |
|---|---|---|---|---|---|---|
| 2.1 | H | H | Methyl | O-Methyl | H | |
| 2.2 | H | H | Methyl | O-Ethyl | H | |
| 2.3 | H | H | Butyl-n | O-Ethyl | H | |
| 2.4 | H | H | Methyl | O-Ethyl | Methyl | |
| 2.5 | H | H | Methyl | S-Ethyl | H | |
| 2.6 | H | H | Propyl-n | S-Propyl-n | H | |
| 2.7 | H | H | Methyl | S-Butyl-n | H | |
| 2.8 | H | H | Methyl | O-Ethyl | Butyl-n | |
| 2.9 | 5-F | H | H | O-Methyl | H | |
| 2.10 | 5-F | H | H | 3-Thienyl | H | Smp. 93- 95°C |
| 2.11 | 5-F | H | H | S-EThyl | H | |
| 2.12 | 6-F | H | H | O-Ethyl | H | |
| 2.13 | 4-F | H | H | O-Ethyl | Propyl-n | |
| 2.14 | 5-F | 6-F | H | O-Ethyl | H | |
| 2.15 | 5-J | H | H | O-Ethyl | H | |
| 2.16 | 5-Br | H | H | O-Ethyl | H | |
| 2.17 | 6-Cl | H | H | O-Ethyl | H | |
| 2.18 | 5-F | H | Methyl | O-Ethyl | H | |
| 2.19 | 5-F | H | Alkyl | O-Ethyl | H | |
| 2.20 | 4-F | 6-F | Butyl | O-Hexyl | Ethyl | |
| 2.21 | 5-F | H | 2-Pentenyl | O-Ethyl | H | |
| 2.22 | 5-F | H | H | 2'-Furyl | H | Smp. 145-147°C |
| 2.23 | 6-F | H | H | 2'-Furyl | H | |
| 2.24 | 4-F | 6-F | H | 2'-Thienyl | H | |
| 2.25 | 5-F | H | H | 3'-Furyl | H | |
| 2.26 | 6-F | H | H | 3'-Thienyl | H | |
| 2.27 | 5-F | 6-F | Allyl | 3'-Furyl | Butyl | |
| 2.28 | 5-F | H | Methyl | 2'-Furyl | H | |
| 2.29 | 4-F | H | H | O-Ethyl | H | |
| 2.30 | 4-F | H | H | 2'-Furyl | H | |
| 2.31 | 4-F | H | H | 3'-Thienyl | H | |
| 2.32 | 5-F | 6-F | H | 3'-Thienyl | H | |

Tabelle 3:

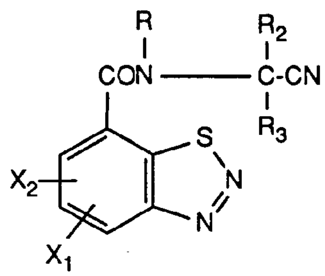

- Ra = $S\text{-}CCl_3$
R - Rb = $S\text{-}CFCl_2$
- Rc = $S\text{-}CCl_2CHCl_2$

| Verb. Nr. | $X_1$ | $X_2$ | R | $R_2$ | $R_3$ | phys. Daten |
|---|---|---|---|---|---|---|
| 3.1 | H | H | Ra | O-Ethyl | H | |
| 3.2 | H | H | Ra | O-Propyl-n | H | |
| 3.3 | 5-F | H | Ra | 2'-Furyl | H | |
| 3.4 | 6-F | H | Ra | 2'-Furyl | H | |
| 3.5 | 5-F | 6-F | Ra | 2'-Thienyl | Methyl | |
| 3.6 | H | H | Ra | 2'-Thienyl | H | |
| 3.7 | H | H | Rb | O-Ethyl | H | |
| 3.8 | H | H | Rb | O-Methyl | H | |
| 3.9 | 5-F | H | Rc | O-Ethyl | H | |
| 3.10 | 6-F | H | Rc | O-Ethyl | H | |
| 3.11 | 4-F | 6-F | Rc | O-Hexyl-n | Butyl-n | |
| 3.12 | H | H | Rc | 2'-Furyl | H | |
| 3.13 | H | H | Rc | 2'-Thienyl | H | |
| 3.14 | H | H | Rb | 2'-Furyl | H | |
| 3.15 | 5-F | H | Rb | 2'-Furyl | Ethyl | |
| 3.16 | 5-Cl | H | Ra | O-Ethyl | H | |
| 3.17 | 5-Br | H | Ra | 3'-Furyl | H | |
| 3.18 | 6-Br | H | Rb | 3'-Thienyl | H | |
| 3.19 | 5-J | H | Rc | 2'-Furyl | H | |
| 3.20 | 6-J | H | Ra | O-Ethyl | Ethyl | |
| 3.21 | H | H | Ra | 3'-Thienyl | H | Smp. 162-164°C |

2. Formulierungsbeispiele für Wirkstoffe der Formel I (% = Gewichtsprozent)

| 2.1 Spritzpulver | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

2.2 Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.3 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

2.4 Extruder Granulat

| | |
|---|---|
| Wirkstoff aus den Tabellen | 10 % |
| Na-Ligninsulfonat | 2 % |
| Carboxymelthylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5 Umhüllungs-Granulat

| | |
|---|---|
| Wirkstoff aus den Tabellen | 3 % |
| Polyethylenglykol (MG 200) | 3 % |
| Kaolin | 94 % |

(MG=Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6 Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Lingninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 200 ppm). Nach 48 Stunden werden die Pflanzen mit einer Sporensuspension (1.5 · $10_5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C bis 23°C weitergeführt.

Die Beurteilung der Schutzwirkung erfolgt aufgrund des Pilzbefalls 7-8 Tage nach der Infektion.

b) Gurkenpflanzen werden nach 2-wöchiger Anzucht mit einer aus Spritzpulver des Wirkstoffes hergestellten Spritzbrühe durch Bodenapplikation behandelt (Konzentration: 60 ppm bezogen auf das Bodenvolumen). Nach 48 Stunden werden die Pflanzen mit einer Sporensuspension (1.5 · $10_5$ Sporen/ml) des Pilzes infiziert und für 36 Stunden bei hoher Luftfeuchtigkeit und einer Temperatur von 23°C inkubiert. Die Inkubation wird dann bei normaler Luftfeuchtigkeit und bei 22°C weitergeführt.

Die Beurteilung der Schutzwirkung erfolgt aufgrund des Pilzbefalls 7-8 Tage nach der Infektion.

Verbindungen aus den Tabellen 1 bis 3 zeigen in den Tests (a) und (b) eine gute Wirkung. So reduzierten z.B. die Verbindungen 1.2 und 1.39 den Pilzbefall auf 0 bis 20 %. Unbehandelte, jedoch infizierte Kontrollpflanzen wiesen dagegen einen Colletotrichum-Befall von 100 % auf.

Beispiel 3.2: Wirkung gegen Phytophthora infestans auf Tomatenpflanzen

a) Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 9O-100 % relativer Luftfeuchtigkeit und 20°C.

b) Zu Tomatenpflanzen wird nach 3-wöchiger Anzucht eine aus Spritzpulver des Wirkstoffes hergestellte Spritzbrühe gegossen (0,006 % Aktivsubstanz bezogen auf das Erdvolumen). Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen 1 bis 3 zeigen gegen den Phytophthora-Pilz eine gute Schutzwirkung. So reduzierte z.B. die Verbindungen 1.2 und 1.39 den Pilzbefall auf 0 bis 20 %. Unbehandelte jedoch infizierte Kontrollpflanzen wiesen dagegen einen 100 %igen Phytophthora-Befall auf.

Beispiel 3.3: Wirkung gegen Plasmopara viticola auf Reben

a) Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.
b) Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20°C werden die infizierten Pflanzen getrocknet und mit einer aus Spritzpulver des Wirkstoffes hergestellten Spritzbrühe (0,06 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen 1 bis 3 zeigen gegen Plasmopara viticola eine gute Schutzwirkung (Befall 0-20 %), so z.B. Verbindung 1.2. Unbehandelte jedoch infizierte Kontrollpflanzen wiesen dagegen einen 100%igen Plasmopara-Befall auf.

Beispiel 3.4: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24°C wird der Pilzbefall beurteilt.
b) Zu 2-wöchigen Reispflanzen wird eine aus Spritzpulver des Wirkstoffes hergestellte Spritzbrühe gegossen (0,006 % Aktivsubstanz bezogen auf das Erdvolumen). Darauf werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95 - 100 % relativer Luftfeuchtigkeit und ca. 24°C wird der Pilzbefall beurteilt.

Reispflanzen, die mit einer Spritzbrühe behandelt werden, die als Aktivsubstanz eine Verbindung aus den Tabellen 1 bis 3 enthält, zeigen im Vergleich zu unbehandelten Kontrollpflanzen (100 % Befall) nur geringen Pilzbefall. So reduzierten z.B. im Test a) die Verbindung 1.39 und im Test b) die Verbindung 1.2 den Befall auf 0 bis 20 %.

3.5 Pythium ultimumauf Beta vulgaris(Zuckerrübe, cv. Kleinwanzleben Monogerm) und auf Pythium ultimumauf Zea mays(Mais, cv. Sweet Corn)

Testprinzip: Bodenpilz; protektiv lokale Bodenapplikation.

Testmethode: Myzel von Pythium ultimum wird mit Erde gemischt (500 ml Myzelsuspension auf 10 Liter Erde) und das Pilz-Erdgemisch in 250 ml Plastikschalen abgefüllt. Nach einer 4-tägigen Inkubation bei 10°C werden pro Schale 10 Körner der zu prüfenden Pflanze (Mais oder Zuckerrübe) gesteckt. Am nächsten Tag werden die so vorbereiteten Schalen mit je 50 ml aus 25 % Spritzpulver und Wasser hergestellten Spritzlösungen mit 20; 6; 2; 0,6; 0,2; 0,06 und 0,02 ppm AS übergossen. Nach einer 7-tägigen Inkubationsphase bei 10°C und einer anschliessenden 4-tägigen Inkubationsphase bei 22°C wird die Wirkung der Testsubstanzen durch zahlenmässige Bestimmung des Auflaufs der Testpflanzen bewertet.

Verbindungen aus den Tabellen 1 bis 3 zeigen gegen Pythium ultimum gute Wirkung. So wies z.B. die Verbindung 1.2 eine Schutzwirkung von mehr als 8O % auf.

Beispiel 3.6: Wirkung gegen Puccinia graminis auf Weizen

a) Weizenpflanzen werden 6 Tage nach der Aussaat mit einer aus Spritzpulver des Wirkstoffes hergestellten Spritzbrühe (0,02 % Aktivsubstanz) besprüht. Nach 24 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.
b) Zu Weizenpflanzen wird 5 Tage nach Aussaat eine aus Spritzpulver des Wirkstoffes hergestellte Spritzbrühe gegossen (0,006 % Aktivsubstanz bezogen auf das Bodenvolumen). Nach 48 Stunden werden die behandelten Pflanzen mit einer Uredosporensuspension des Pilzes infiziert. Nach einer Inkubation während 48 Stunden bei 95-100 % relativer Luftfeuchtigkeit und ca. 20°C werden die infizierten Pflanzen in einem Gewächshaus bei ca. 22°C aufgestellt. Die Beurteilung der Rostpustelnentwicklung erfolgt 12 Tage nach der Infektion.

Verbindungen aus den Tabellen 1 bis 3 zeigen gegen Puccinia-Pilze eine gute Wirkung. So reduzierte z.B. die Verbindung 1.2 den Pilzbefall auf 0 bis 20 %. Unbehandelte jedoch infizierte Kontrollpflanzen wiesen dagegen einen Puccinia-Befall von 100 % auf.

Beispiel 3.7: Wirkung gegen Erysiphe graminis auf Weizen

Saatweizen der Sorte Bernina wurde Ende September 1988 auf Prüfparzellen der Teststation St.Aubin, Fribourg, Schweiz, ausgesät. In der ersten Aprilwoche 1989 wurden die Pflanzen bei einer durchschnittlichen Höhe von ca. 20 cm und einem maximalen natürlichen Krankheitsbefall von 1 % durch Erysiphe graminis mit einer wässrigen Suspension der Wirksubstanz in einer Konzentration von 125 g in 500 l Wasser pro Hektar durch Besprühen des Blattwerks behandelt.

Die Beurteilung der Schutzwirkung wurde aufgrund des Pilzbefalls nach 55 Tagen nach der Behandlung vorgenommen. Als Wirkstoff eingesetzte Verbindungen aus den Tabellen 1 bis 3 zeigten eine gute Wirkung gegen Erysiphe graminis. So blieben Pflanzen, die z.B. mit der Verbindung 1.2 behandelt wurden, weitgehend frei von Erysiphe-Befall (0-15 % Schädigung). Unbehandelte, unter gleichen Bedingungen erkrankte Kontrollpflanzen wiesen dagegen einen Befall von 35 % auf.

Beispiel 3.8: Wirkung gegen Peronospora tabacina an Takbakpflanzen Blattapplikation

Tabakpflanzen (8 Wochen alt) werden mit einer formulierten Lösung des Wirkungsstoffes besprüht (Konzentration: 0,02 % Aktivsubstanz). Vier Tage nach der Behandlung werden die Pflanzen mit einer Sporangiensuspension von Peronospora tabacina ($10^4$ Sporangien/ml) inokuliert, 20 Stunden im Dunkeln bei 25°C und hoher Luftfeuchtigkeit aufbewahrt und dann bei normaler Tag/Nacht Wechselfolge weiterinkubiert.

Die Beurteilung der Symptome in den Tests erfolgt aufgrund der mit Pilz befallenen Blattoberfläche. Pflanzen, welche im Test z.B. mit der Verbindung 1.2 behandelt wurden, zeigten einen Befall von 0-35 %. Die Kontrollpflanzen wiesen dagegen einen Befall von 90 bis 100 % auf.

Beispiel 3.9: Wirkung gegen Bremia letucae auf Salat

In Petrischalen enthaltend je 100 $cm^3$ steriles Vermikulit (exfolierter Glimmer; Fa. Vermica AG, Bözen, CH) werden je 80 ml einer Testsuspension mit einem Gehalt von 10 ppm Wirksubstanz gegeben. Dann werden 50 Salatsamen je Petrischale auf die Oberfläche der behandelten Vermikulitschicht gebracht und die Petrischalen bei 21°C und einem Tag/Nacht-Rhythmus von je 12 Stunden für ca. 7 Tage inkubiert. Die sich entwickelnden Pflanzen werden dann mit einer versprühten Sporensuspension (Konzentration 5 x $10^4$ Sporen pro ml) inokuliert. Danach werden die so präparierten Petrischalen im Dunkeln vei 16°C für 18-24 Stunden inkubiert. Anschliessend wird die Inkubation im 12-Stunden-Tageslichtrhythmus bei gedämpfter Beleuchtung fortgesetzt. 2-3 Wochen nach der Infektion wird die Wirkung durch Auszählung der infizierten Sämlinge im Vergleich mit den nicht-medikierten Kontrollschalen ermittelt.

Verbindungen aus den Tabellen 1 bis 3 zeigen eine gute Wirkung gegen Bremia. So waren z.B. nach Behandlung mit der Verbindung 1.2 oder 1.39 weniger als 20 % der Sämiinge befallen. In den unbehandelten, jedoch infizierten Kontrollschalen betrug der Befall dagegen mehr als 90 %.

## Patentansprüche

### Patentansprüche für folgende Vertragsstaaten : AT, BE, CH, DE, DK, FR, GB, GR, IT, LI, LU, NL, SE

1. Verbindungen der Formel I

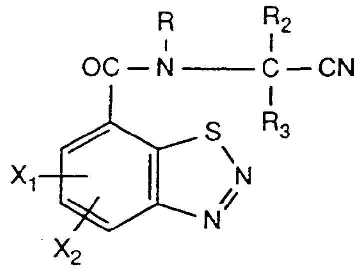

(I)

in welcher bedeuten:

$X_1$ und $X_2$ unabhängig voneinander Wasserstoff oder Halogen;
R Wasserstoff, $C_1$-$C_4$-Alkyl, $C_3$-$C_5$-Alkenyl oder $SR_1$;
$R_1$ mit 3 bis 6 Fluor oder Chlor substituiertes $C_1$-$C_3$-Alkyl;
$R_2$ $C_1$-$C_4$-Alkyl, durch Sauerstoff unterbrochenes $C_1$-$C_5$-Alkyl, $C_1$-$C_6$-Alkoxy, mit 1 bis 3 Halogen substituiertes $C_1$-$C_3$-Alkyl oder substituiertes $C_2$-$C_3$-Alkoxy, $C_1$-$C_6$-Alkylthio, in 2- oder 3-Stellung gebundenes Furyl oder gebundenes Thienyl, mit 1 bis 3 Halogen substituiertes und in 2- oder 3-Stellung gebundenes Furyl oder gebundenes Thienyl; und
$R_3$ Wasserstoff oder $C_1$-$C_4$-Alkyl.

**2.** Verbindungen der Formel I, worin bedeuten:

$X_1$ und $X_2$ unabhängig voneinander Wasserstoff oder Fluor;
R Wasserstoff, Methyl, Ethyl, Allyl oder den Rest S-$R_1$;
$R_1$ Trichlormethyl, Dichlorfluormethyl oder 1,1,2,2-Tetrachlorethyl.

**3.** Verbindungen der Formel I, worin bedeuten:

$X_1$ und $X_2$ unabhängig voneinander Wasserstoff oder Fluor;
R Wasserstoff;
$R_2$ $C_1$-$C_4$-Alkoxy, $C_1$-$C_4$-Alkylthio, in 2- oder 3-Stellung gebundenes Furyl oder Thienyl, mit 1 bis 3 Halogen substituiertes und in 2- oder 3-Stellung gebundenes Furyl oder gebundenes Thienyl; und
$R_3$ Wasserstoff oder $C_1$-$C_3$-Alkyl.

**4.** Verbindungen der Formel I gemäss Anspruch 1, worin bedeuten:

$X_1$ und $X_2$ unabhängig voneinander Wasserstoff oder Fluor; R Wasserstoff, Methyl, Ethyl, Allyl oder den Rest S-$R_1$;
$R_1$ Trichlormethyl, Dichlorfluormethyl oder 1,1,2,2-Tetrachlorethyl;
$R_2$ $C_1$-$C_4$-Alkyl, durch Sauerstoff unterbrochenes $C_1$-$C_5$-Alkyl, $C_1$-$C_6$-Alkoxy, Trichlormethyl, Trifluormethyl, 1,1,1-Trifluorethoxy, $C_1$-$C_6$-Alkylthio, in 2- oder 3-Stellung gebundenes Furyl oder gebundenes Thienyl, mit 1 bis 3 Halogen substituiertes und in 2- oder 3-Stellung gebundenes Furyl oder gebundenes Thienyl; und
$R_3$ Wasserstoff oder Methyl.

**5.** Verbindungen der Formel I gemäss Anspruch 1, worin bedeuten:

$X_1$ und $X_2$ unabhängig voneinander Wasserstoff oder Fluor;
R Wasserstoff, Methyl, Ethyl, Allyl oder den Rest S-$R_1$;
$R_1$ Trichlormethyl, Dichlorfluormethyl oder 1,1,2,2-Tetrachlorethyl;
$R_2$ $C_1$-$C_4$-Alkyl, durch Sauerstoff unterbrochenes $C_1$-$C_5$-Alkyl, $C_1$-$C_6$-Alkoxy, Trichlormethyl, Trifluormethyl, 1,1,1-Trifluorethoxy, $C_1$-$C_6$-Alkylthio, in 2- oder 3-Stellung gebundenes Furyl oder Thienyl, mit 1 bis 3 Halogen substituiertes und in 2- oder 3-Stellung gebundenes Furyl oder gebundenes Thienyl; und
$R_3$ Wasserstoff.

**6.** Verbindungen der Formel I gemäss Anspruch 1, worin bedeuten:

$X_1$ und $X_2$ unabhängig voneinander Wasserstoff oder Fluor;
R Wasserstoff;
$R_2$ $C_1$-$C_6$-Alkoxy, $C_1$-$C_6$-Alkylthio oder in 2- oder 3-Stellung gebundenes Furyl oder gebundenes Thienyl; und

$R_3$ Wasserstoff.

7. Eine Verbindung gemäss Anspruch 1 aus der Gruppe:
N-(Benz-1,2,3-thiadiazol-7-carbonyl)-2-ethoxy-2-aminoacetonitril;
N-(Benz-1,2,3-thiadiazol-7-carbonyl)-2-methoxy-2-aminoacetonitril;
N-(Benz-1,2,3-thiadiazol-7-carbonyl)-2-(furyl-2′yl)-2-aminoacetonitril;
N-(Benz-1,2,3-thiadiazol-7-carbonyl)-2-(thiophen-2′yl)-2-aminoacetonitril;
N-(Benz-1,2,3-thiadiazol-7-carbonyl)-2-(thiophen-3′yl)-2-aminoacetonitril;
N-(5-Fluor-benz-1,2,3-thiadiazol-7-carbonyl)-2-(thiophen-3′yl)-2-aminoacetonitril;
N-(4-Fluor-benz-1,2,3-thiadiazol-7-carbonyl)-2-(thiophen-3′yl)-2-aminoacetonitril;
N-(Benz-1,2,3-thiadiazol-7-carbonyl)-2-(furyl-3′yl)-2-aminoacetonitril;
N-(Benz-1,2,3-thiadiazol-7-carbonyl)2-methylthio-2-aminoacetonitril;
N-(Benz-1,2,3-thiadiazol-7-carbonyl)-2-isopropylthio-2-aminoacetonitril.

8. Verfahren zur Herstellung von Verbindungen der Formel I in Anspruch 1 mit Ausnahme derjenigen Verbindungen, in denen R für den Rest S-$R_1$ und $R_2$ für $C_1$-$C_6$-Alkoxy oder $C_1$-$C_6$-Alkylthio stehen, indem man umsetzt: eine Benzthiadiazolverbindung der Formel II

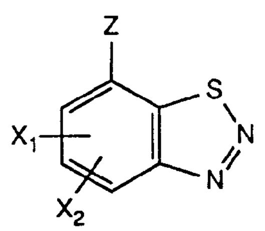

(II)

mit einer Aminoverbindung der Formel III

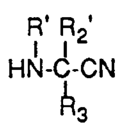

(III)

in Gegenwart einer Base und gegebenenfalls mit einem Katalysator in inerten Lösungsmitteln bei Temperaturen von -10° bis 160°C, wobei Z die Reste COOH, Hal-CO, COOC$_1$-C$_5$-Alkyl,

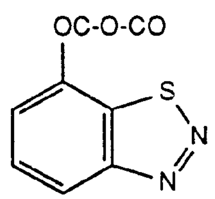

oder

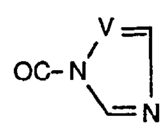

bedeutet, V CH oder N und Hal Halogen darstellen und R′ die Bedeutung von R unter Formel I mit Ausnahme von S-$R_1$ und $R_2'$ die Bedeutung von $R_2$ unter Formel I mit Ausnahme von $C_1$-$C_6$-Alkoxy oder $C_1$-$C_6$-Alkylthio haben und $X_1$, $X_2$ und $R_3$ die unter der Formel I angegebenen Bedeutungen besitzen.

9. Verfahren zur Herstellung von Verbindungen der Formel I in Anspruch 1, in denen für R die Bedeutung S-$R_1$ ausgeschlossen ist (=R′) und in welchen $R_2$ $C_1$-$C_6$-Alkoxy oder $C_1$-$C_6$-Alkylthio (=$R_2''$) bedeutet, indem man:
a) eine Benzthiadiazolverbindung der Formel II

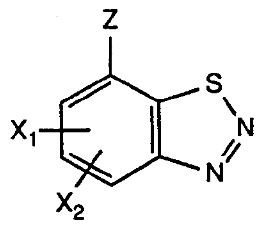

(II)

mit einer Aminoverbindung der Formel III'

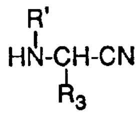

(III')

in Gegenwart einer Base in inerten Lösungsmitteln bei Temperaturen von -10° bis 160°C zu einer Amidverbindung der Formel IV

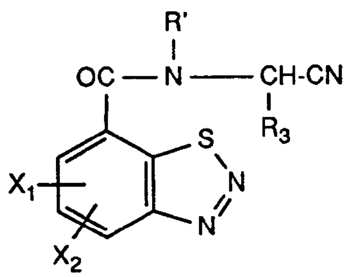

(IV)

umsetzt und anschliessend

b) die Verbindung der Formel IV mit einem Halogenierungsmittel in inerten Lösungsmitteln bei Temperaturen von -30° bis 70°C in eine Halogenverbindung der Formel V

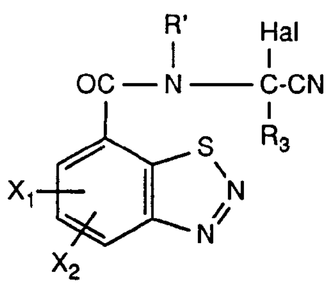

(V)

überführt und dann

c) die Verbindung der Formel V nach Isolierung oder ohne Isolierung mit einem Alkohol oder Thiol der Formel VI

$HR_2''$ (VI)

in Gegenwart einer Base in inerten Lösungsmitteln bei Temperaturen von -20° bis 160°C verethert, wobei Z die Reste COOH, Hal-CO, $COOC_1$-$C_3$-Alkyl,

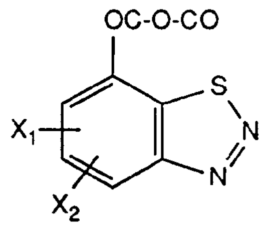

oder

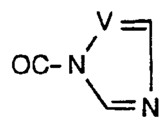

bedeutet, V CH oder N und Hal Halogen darstellen und R′ die Bedeutung von R unter Formel I mit Ausnahme von S-$R_1$ hat und $R_2''$ die Bedeutung von $C_1$-$C_6$-Alkoxy und $C_1$-$C_6$-Alkylthio hat und $X_1$, $X_2$ und $R_3$ die unter Formel I angegebenen Bedeutungen besitzt.

**10.** Verfahren zur Herstellung von Verbindungen der Formel I in Anspruch 1, in welcher R die Bedeutung von S-$R_1$ hat indem man eine Verbindung der Formel Ia

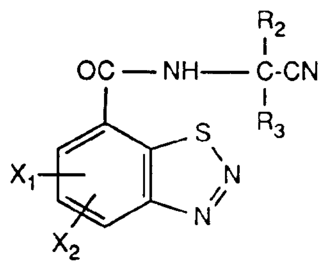

(Ia)

mit einem Sulfenylhalogenid der Formel VII

$$\text{Hal-S-}R_1 \qquad \text{(VII)}$$

in Gegenwart einer Base in inerten Lösungsmitteln bei Temperaturen von -10° bis 80°C umsetzt, wobei Hal Halogen bedeutet und $X_1$, $X_2$, $R_1$, $R_2$ und $R_3$ die unter der Formel I angegebenen Bedeutungen besitzt.

**11.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**12.** Mittel gemäss Anspruch 11, dadurch gekennzeichnet, dass es als aktive Komponente mindestens eine Verbindung gemäss den Ansprüchen 2 bis 6 enthält.

**13.** Mittel gemäss Anspruch 11, dadurch gekennzeichnet, dass es als aktive Komponente die Verbindung der Formel I gemäss Anspruch 7 enthält.

**14.** Verfahren zur Herstellung eines agrochemischen Mittels von Anspruch 11, dadurch gekennzeichnet, dass man mindestens eine gemäss Anspruch 1 definierte Verbindung mit geeigneten festen oder flüssigen Träger- und Hilfsstoffen innig vermischt.

**15.** Verwendung von Verbindungen gemäss Anspruch 1 zum Schutz von Pflanzen gegen den Befall durch phytopathogene Mikroorganismen.

**16.** Verwendung von Verbindungen gemäss einem der Ansprüche 2 bis 7 zum Schutz von Pflanzen gegen den Befall durch phytopathogene Mikroorganismen.

**17.** Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

**18.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 7 auf die Pflanze oder deren Standort appliziert.

**19.** Verfahren gemäss den Ansprüchen 17 und 18, dadurch gekennzeichnet, dass es sich bei den phytopathogenen Mikroorganismen um Pilzorganismen handelt.

**Patentansprüche für folgende Vertragsstaat : ES**

**1.** Verfahren zur Herstellung der Formel I

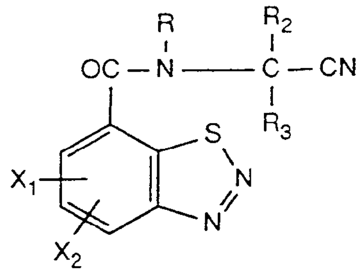

(I)

in welcher bedeuten:

| | |
|---|---|
| $X_1$ und $X_2$ | unabhängig voneinander Wasserstoff oder Halogen; |
| R | Wasserstoff, $C_1$-$C_4$-Alkyl, $C_3$-$C_5$-Alkenyl oder $SR_1$; |
| $R_1$ | mit 3 bis 6 Fluor oder Chlor substituiertes $C_1$-$C_3$-Alkyl; |
| $R_2$ | $C_1$-$C_4$-Alkyl, durch Sauerstoff unterbrochenes $C_1$-$C_5$-Alkyl, $C_1$-$C_6$-Alkoxy, mit 1 bis 3 Halogen substituiertes $C_1$-$C_3$-Alkyl oder substituiertes $C_2$-$C_3$-Alkoxy, $C_1$-$C_6$-Alkylthio, in 2- oder 3-Steflung gebundenes Furyl oder gebundenes Thienyl, mit 1 bis 3 Halogen substituiertes und in 2- oder 3-Stellung gebundenes Furyl oder gebundenes Thienyl; und |
| $R_3$ | Wasserstoff oder $C_1$-$C_4$-Alkyl; |

mit Ausnahme derjenigen Verbindungen, in denen R für den Rest S-$R_1$ und $R_2$ für $C_1$-$C_6$-Alkoxy oder $C_1$-$C_6$-Alkylthio stehen, indem man umsetzt: eine Benzthiadiazolverbindung der Formel II

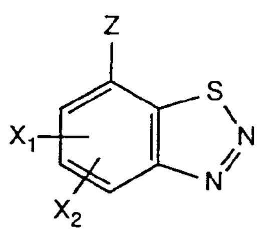

(II)

mit einer Aminoverbindung der Formel III

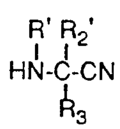

(III)

in Gegenwart einer Base und gegebenenfalls mit einem Katalysator in inerten Lösungs- mitteln bei Temperaturen von -10° bis 160°C, wobei Z die Reste COOH, Hal-CO, COO$C_1$-$C_5$-Alkyl,

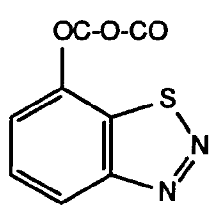

oder

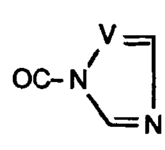

bedeutet, V CH oder N und Hal Halogen darstellen und R′ die Bedeutung von R unter Formel I mit Aus-

nahme von S-$R_1$ und $R_2'$ die Bedeutung von $R_2$ unter Formel I mit Ausnahme von $C_1$-$C_6$-Alkoxy oder $C_1$-$C_6$-Alkylthio haben und $X_1$, $X_2$ und $R_3$ die unter der Formel I angegebenen Bedeutungen besitzen.

**2.** Verfahren zur Herstellung von Verbindungen der Formel I in Anspruch 1, in denen für R die Bedeutung S-$R_1$ ausgeschlossen ist (=R′) und in welchen $R_2$ $C_1$-$C_6$-Alkoxy oder $C_1$-$C_6$-Alkylthio (=$R_2''$) bedeutet, indem man:

a) eine Benzthiadiazolverbindung der Formel II

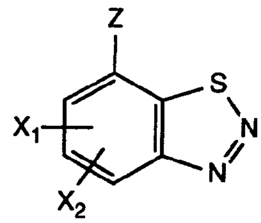

(II)

mit einer Aminoverbindung der Formel III′

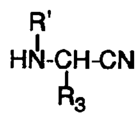

(III')

in Gegenwart einer Base in inerten Lösungsmitteln bei Temperaturen von -10° bis 160°C zu einer Amidverbindung der Formel IV

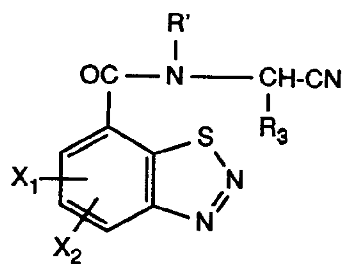

(IV)

umsetzt und anschliessend

b) die Verbindung der Formel IV mit einem Halogenierungsmittel in inerten Lösungs- mitteln bei Temperaturen von -30° bis 70°C in eine Halogenverbindung der Formel V

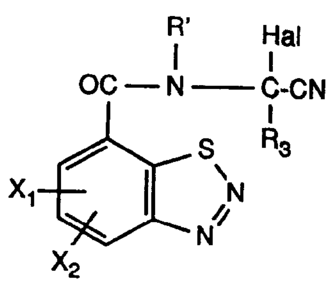

(V)

überführt und dann

c) die Verbindung der Formel V nach Isolierung oder ohne Isolierung mit einem Alkohol oder Thiol der Formel VI

$HR_2''$ (VI)

in Gegenwart einer Base in inerten Lösungsmitteln bei Temperaturen von -20° bis 160°C verethert, wobei Z die Reste COOH,Hal-CO, COOC$_1$-C$_3$-Alkyl,

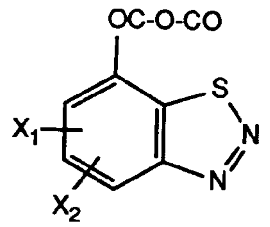

oder

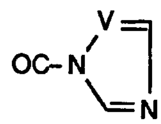

bedeutet, V CH oder N und Hal Halogen darstellen und R′ die Bedeutung von R unter Formel I mit Ausnahme von S-R$_1$ hat und R$_2$″ die Bedeutung von C$_1$-C$_6$-Alkoxy und C$_1$-C$_6$-Alkynlthio hat und X$_1$, X$_2$ und R$_3$ die unter Formel I angegebenen Bedeutungen besitzt.

3. Verfahren zur Herstellung von Verbindungen der Formel I in Anspruch 1, in welcher R die Bedeutung von S-R$_1$ hat indem man eine Verbindung der Formel Ia

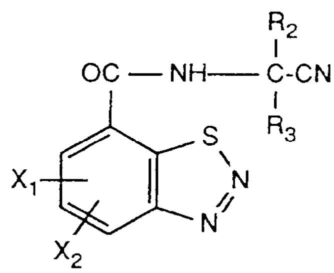

(Ia)

mit einem Sulfenylhalogenid der Formel VII

Hal-S-R$_1$ (VII)

in Gegenwart einer Base in inerten Lösungsmitteln bei Temperaturen von -10° bis 80°C umsetzt, wobei Hal Halogen bedeutet und X$_1$, X$_2$, R$_1$, R$_2$ und R$_3$ die unter der Formel I angegebenen Bedeutungen besitzt.

4. Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung von Anspruch 1 enthält.

5. Mittel gemäss Anspruch 4, dadurch gekennzeichnet, dass es als aktive Komponente mindestens eine Verbindung von Anspruch 2 oder 3 enthält.

6. Mittel gemäss Anspruch 4, dadurch gekennzeichnet, dass es als aktive Komponente mindestens eine Verbindung der Formel I von Anspruch 1 enthält, worin bedeuten:

| | |
|---|---|
| X$_1$ und X$_2$ | unabhängig voneinander Wasserstoff oder Fluor: |
| R | Wasserstoff, Methyl, Ethyl, Allyl oder den Rest S-R$_1$; |
| R$_1$ | Trichlormethyl, Dichlorfluormethyl oder 1,1,2,2-Tetrachlorethyl. |

7. Mittel gemäss Anspruch 4, dadurch gekennzeichnet, dass es als aktive Komponente mindestens eine Verbindung der Formel I von Anspruch 1 enthält, worin bedeuten:

| | |
|---|---|
| X$_1$ und X$_2$ | unabhängig voneinander Wasserstoff oder Fluor, |
| R | Wasserstoff; |
| R$_2$ | C$_1$-C$_4$-Alkoxy, C$_1$-C$_4$-Alkylthio, in 2- oder 3-Stellung gebundenes Furyl oder Thienyl, mit 1 bis 3 Halogen substituiertes und in 2- oder 3-Stellung gebundenes Furyl oder gebundenes Thienyl; und |
| R$_3$ | Wasserstoff oder C$_1$-C$_3$-Alkyl. |

8. Mittel gemäss Anspruch 4, dadurch gekennzeichnet, dass es als aktive Komponente mindestens eine Verbindung der Formel I von Anspruch 1 enthält, worin bedeuten:

$X_1$ und $X_2$ unabhängig voneinander Wasserstoff oder Fluor, R Wasserstoff, Methyl, Ethyl, Allyl oder den Rest $S-R_1$;
$R_1$ Trichlormethyl, Dichlorfluormethyl oder 1,1,2,2;
$R_2$ $C_1$-$C_4$-Alkyl, durch Sauerstoff unterbrochenes $C_1$-$C_5$-Alkyl, $C_1$-$C_6$-Alkoxy, Trichlor- methyl, Trifluormethyl, 1,1,1-Trifluorethoxy, $C_1$-$C_6$-Alkylthio, in 2- oder 3-Stellung gebundenes Furyl oder gebundenes Thienyl, mit 1 bis 3 Halogen substituiertes und in 2- oder 3-Stellung gebundenes Furyl oder gebundenes Thienyl; und
$R_3$ Wasserstoff oder Methyl.

**9.** Mittel gemäss Anspruch 4, dadurch gekennzeichnet, dass es als aktive Komponente mindestens eine Verbindung der Formel I von Anspruch 1 enthält, worin bedeuten:
$X_1$ und $X_2$ unabhängig voneinander Wasserstoff oder Fluor;
R Wasserstoff, Methyl, Ethyl, Allyl oder den Rest $S-R_1$,
$R_1$ Trichlormethyl, Dichlorfluormethyl oder 1,1,2,2-Tetrachlorethyl;
$R_2$ $C_1$-$C_4$-Alkyl, durch Sauerstoff unterbrochenes $C_1$-$C_5$-Alkyl, $C_1$-$C_6$-Alkoxy, Trichlor- methyl, Trifluormethyl, 1,1,1-Trifiuorethoxy, $C_1$-$C_6$-Alkylthio, in 2- oder 3-Stellung gebundenes Furyl oder Thienyl, mit 1 bis 3 Halogen substituiertes und in 2- oder 3-Stellung gebundenes Furyl oder gebundenes Thienyl; und
$R_3$ Wasserstoff.

**10.** Mittel gemäss Anspruch 4, dadurch gekennzeichnet, dass es als aktive Komponente mindestens eine Verbindung der Formel I von Anspruch 1 enthält, worin bedeuten:
$X_1$ und $X_2$ unabhängig voneinander Wasserstoff oder Fluor,
R Wasserstoff;
$R_2$ $C_1$-$C_6$-Alkoxy, $C_1$-$C_6$-Alkylthio oder in 2- oder 3-Stellung gebundenes Furyl oder gebundenes Thienyl; und
$R_3$ Wasserstoff.

**11.** Mittel gemäss Anspruch 4, dadurch gekennzeichnet, dass es als aktive Komponente mindestens eine Verbindung aus der Gruppe:
N-(Benz-1,2,3-thiadiazol-7-carbonyl)-2-ethoxy-2-aminoacetonitril;
N-(Benz-1,2,3-thiadiazol-7-carbonyl)-2-aminoacetonitril;
N-(Benz-1,2,3-thiadiazol-7-carbonyl)-2-(furyl-2'yl)-2-aminoacetonitril;
N-(Benz-1,2,3-thiadiazol-7-carbonyl)-2-(thiophen-2'yl)-2-aminoacetonitril;
N-(Benz-1,2,3-thiadiazol-7-carbonyl)-2-(thiophen-3'yl)-2-aminoacetonitril;
N-(5-Fluor-benz-1,2,3-thiadiazol-7-carbonyl)-2-(thiophen-3'yl)-2-aminoacetonitril;
N-(4-Fluor-benz-1,2,3-thiadiazol-7-carbonyl)-2-(thiophen-3'yl)-2-aminoacetonitril;
N-(Benz-1,2,3-thiadiazol-7-carbonyl)-2-(furyl-3'yl)-2-aminoacetonitril;
N-(Benz-1,2,3-thiadiazol-7-carbonyl)-2-methylthio-2-aminoacetonitril;
N-(Benz-1,2,3-thiadiazol-7-carbonyl)-2-isopropylthio-2-aminoacetonitril, enthält.

## Claims

### Claims for the following Contracting States : AT, BE, CH, DE, DK, FR, GB, GR, IT, LI, LU, NL, SE

**1.** A compound of the formula I

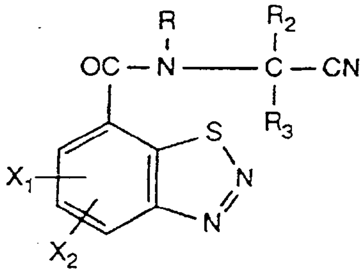

(I)

in which:
$X_1$ and $X_2$ independently of one another are hydrogen or halogen; R is hydrogen, $C_1$-$C_4$alkyl, $C_3$-$C_5$alkenyl or $SR_1$; $R_1$ is $C_1$-$C_3$alkyl which is substituted by 3 to 6 fluorine or chlorine; $R_2$ is $C_1$-$C_4$alkyl, or is $C_1$-$C_5$alkyl which is interrupted by oxygen, or is $C_1$-$C_6$alkoxy, or is $C_1$-$C_3$alkyl which is substituted by 1 to 3 halogen, or is substituted $C_2$-$C_3$alkoxy, or is $C_1$-$C_6$alkylthio, or is furyl or thienyl, each of which is bonded in the 2- or 3-position, or is furyl or thienyl, each of which is substituted by 1 to 3 halogen and bonded in the 2- or 3-position; and $R_3$ is hydrogen or $C_1$-$C_4$alkyl.

2. A compound of the formula I where: $X_1$ and $X_2$ independently of one another are hydrogen or fluorine; R is hydrogen, methyl, ethyl, allyl or the radical S-$R_1$; $R_1$ is trichloromethyl, dichlorofluoromethyl or 1,1,2,2-tetrachloroethyl.

3. A compound of the formula I where: $X_1$ and $X_2$ independently of one another are hydrogen or fluorine; R is hydrogen; $R_2$ is $C_1$-$C_4$alkoxy, $C_1$-$C_4$alkylthio, or is furyl or thienyl, each of which is bonded in the 2- or 3-position, or is furyl or thienyl, each of which is substituted by 1 to 3 halogen and bonded in the 2- or 3-position; and $R_3$ is hydrogen or $C_1$-$C_3$alkyl.

4. A compound of the formula I according to claim 1, where: $X_1$ and $X_2$ independently of one another are hydrogen or fluorine; R is hydrogen, methyl, ethyl, allyl or the radical S-$R_1$; $R_1$ is trichloromethyl, dichlorofluoromethyl or 1,1,2,2-tetrachloroethyl; $R_2$ is $C_1$-$C_4$alkyl, or $C_1$-$C_5$alkyl which is interrupted by oxygen, or $C_1$-$C_6$alkoxy, trichloromethyl, trifluoromethyl, 1,1,1-trifluoroethoxy, $C_1$-$C_6$alkylthio, or is furyl or thienyl, each of which is bonded in the 2- or 3-position, or is furyl or thienyl, each of which is substituted by 1 to 3 halogen and bonded in the 2- or 3-position; and $R_3$ is hydrogen or methyl.

5. A compound of the formula 1 according to claim 1, where: $X_1$ and $X_2$ independently of one another are hydrogen or fluorine; R is hydrogen, methyl, ethyl, allyl or the radical S-$R_1$; $R_1$ is trichloromethyl, dichlorofluoromethyl or 1,1,2,2-tetrachloroethyl; $R_2$ is $C_1$-$C_4$alkyl, or is $C_1$-$C_5$alkyl which is interrupted by oxygen, or is $C_1$-$C_6$alkoxy, trichloromethyl, trifluoromethyl, 1,1,1-trifluoroethoxy, $C_1$-$C_6$alkylthio, or is furyl or thienyl, each of which is bonded in the 2- or 3-position, or is furyl or thienyl, each of which is substituted by 1 to 3 halogen and bonded in the 2- or 3-position; and $R_3$ is hydrogen.

6. A compound of the formula I according to claim 1, where: $X_1$ and $X_2$ independently of one another are hydrogen or fluorine; R is hydrogen; $R_2$ is $C_1$-$C_6$alkoxy, $C_1$-$C_6$alkylthio, or is furyl or thienyl, each of which is bonded in the 2- or 3-position; and $R_3$ is hydrogen.

7. A compound according to claim 1 from the group comprising:
N-(benzo-1,2,3-thiadiazole-7-carbonyl)-2-ethoxy-2-aminoacetonitrile;
N-(benzo-1,2,3-thiadiazole-7-carbonyl)-2-methoxy-2-aminoacetonitrile;
N-(benzo-1,2,3-thiadiazole-7-carbonyl)-2-(fur-2′-yl)-2-aminoacetonitrile;
N-(benzo-1,2,3-thiadiazole-7-carbonyl)-2-(thiophen-2′-yl)-2-aminoacetonitrile;
N-(benzo-1,2,3-thiadiazole-7-carbonyl)-2-(thiophen-3′-yl)-2-aminoacetonitrile;
N-(5-fluorobenzo-1,2,3-thiadiazole-7-carbonyl)-2-(thiophen-3′-yl)-2-aminoacetonitrile;
N-(4-fluorobenzo-1,2,3-thiadiazole-7-carbonyl)-2-(thiophen-3′-yl)-2-aminoacetonitrile;
N-(benzo-1,2,3-thiadiazole-7-carbonyl)-2-(fur-3′-yl)-2-aminoacetonitrile;
N-(benzo-1,2,3-thiadiazole-7-carbonyl)-2-methylthio-2-aminoacetonirile;
N-(benzo-1,2,3-thiadiazole-7-carbonyl)-2-isopropylthio-2-aminoacetonitrile.

8. A process for the preparation of compounds of the formula I of claim 1, with the exception of those compounds in which R is the radical S-$R_1$ and $R_2$ is $C_1$-$C_6$alkoxy or $C_1$-$C_6$alkylthio, by reacting: a benzothiadiazole compound of the formula II

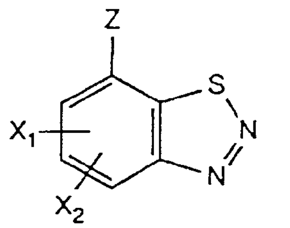

(II)

with an amino compound of the formula III

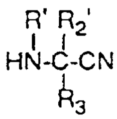

(III)

in the presence of a base and if appropriate with a catalyst, in inert solvents at temperatures of from -10° to 160°C, where Z is the radicals COOH, Hal-CO, $COOC_1$-$C_5$alkyl,

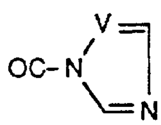

or

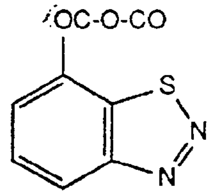

V is CH or N and Hal is halogen and R′ has the meaning of R as in formula I with the exception of S-$R_1$, and $R_2'$ has the meaning of $R_2$ as in formula I, with the exception of $C_1$-$C_6$alkoxy or $C_1$-$C_6$alkylthio, and $X_1$, $X_2$ and $R_3$ have the meanings indicated in the case of formula I.

**9.** A process for the preparation of compounds of the formula I in claim 1, in which the meaning S-$R_1$ is excluded for R (=R′) and in which $R_2$ is $C_1$-$C_6$alkoxy or $C_1$-$C_6$alkylthio (=$R_2''$), by:
a) reacting a benzothiadiazole compound of the formula II

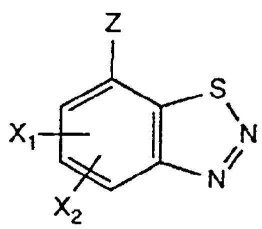

(II)

with an amino compound of the formula III′

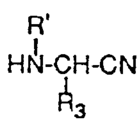

(III′)

in the presence of a base in inert solvents at temperatures of from -10°C to 160°C to give an amide compound of the formula IV

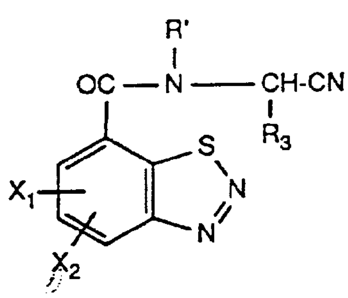

(IV)

and subsequently

b) converting the compound of the formula IV into a halogen compound of the formula V

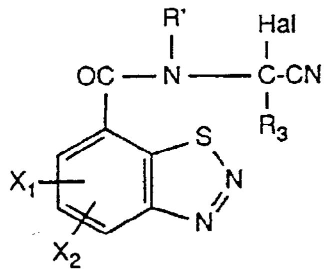

(V)

with a halogenating agent in inert solvents at temperatures of from - 30°C to 70°C and then

c) etherifying the compound of the formula V, after isolation or without isolation, with an alcohol or thiol of the formula VI

$HR_2''$ (VI)

in the presence of a base in inert solvents at temperatures of from -20°C to 160°C, where Z is the radicals COOH, Hal-CO, $COOC_1$-$C_3$alkyl,

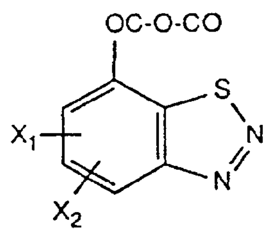

or

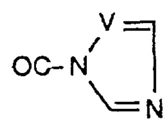

V is CH or N and Hal is halogen, and R′ has the meaning of R as in formula I with the exception of S-$R_1$, and $R_2''$ has the meaning of $C_1$-$C_6$alkoxy and $C_1$-$C_6$alkylthio, and $x_1$, $x_2$ and $R_3$ have the meanings indicated in the case of formula I.

**10.** Process for the preparation of compounds of the formula I in claim 1, in which R has the meaning of S-$R_1$, by reacting a compound of the formula Ia

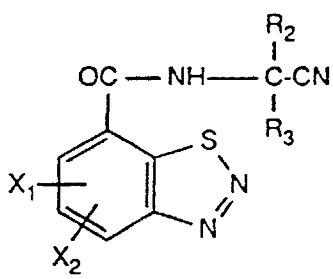

(Ia)

with a sulfenyl halide of the formula VII

Hal-S-$R_1$ (VII)

in the presence of a base in inert solvents at temperatures of from -10° to 80°C, where Hal is halogen and $X_1$, $X_2$, $R_1$, $R_2$ and $R_3$ have the meanings indicated in the case of the formula I.

**11.** An agent for protecting plants against attack by microorganisms, which contains, besides customary carriers and auxiliary substances, at least one compound according to claim 1 as the active component.

**12.** An agent according to claim 11, which contains at least one compound according to claims 2 to 6 as the active component.

**13.** An agent according to claim 11, which contains a compound of the formula I according to claim 7 as the

active component.

**14.** A process for the preparation of an agrochemical of claim 11, which comprises mixing intimately at least one compound as defined according to claim 1 with suitable solid or liquid carriers and auxiliary substances.

**15.** The use of a compound according to claim 1 for protecting plants against attack by phytopathogenic microorganisms.

**16.** The use of a compound according to any one of claims 2 to 7 for protecting plants against attack by phytopathogenic microorganisms.

**17.** A method of protecting plants against attack by phytopathogenic microorganisms, which comprises applying, as the active ingredient, a compound according to claim 1 to the plant or its habitat.

**18.** A method of protecting plants against attack by phytopathogenic microorganisms, which comprises applying, as the active ingredient, a compound according to any one of claims 2 to 7 to the plant or its habitat.

**19.** A method according to claims 17 and 18, in which the phytopathogenic microorganisms are fungal organisms.

**Claims for the following Contracting State : ES**

**1.** A process for the preparation of a compound of the formula I

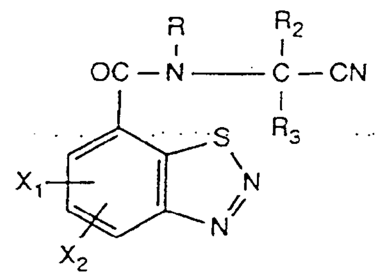

(I)

in which:

$X_1$ and $X_2$ independently of one another are hydrogen or halogen; R is hydrogen, $C_1$-$C_4$alkyl, $C_3$-$C_5$alkenyl or $SR_1$; $R_1$ is $C_1$-$C_3$alkyl which is substituted by 3 to 6 fluorine or chlorine; $R_2$ is $C_1$-$C_4$alkyl, or is $C_1$-$C_5$alkyl which is interrupted by oxygen, or is $C_1$-$C_6$alkoxy, or is $C_1$-$C_3$alkyl which is substituted by 1 to 3 halogen, or is substituted $C_2$-$C_3$alkoxy, or is $C_1$-$C_6$alkylthio, or is furyl or thienyl, each of which is bonded in the 2- or 3-position, or is furyl or thienyl, each of which is substituted by 1 to 3 halogen and bonded in the 2- or 3-position; and $R_3$ is hydrogen or $C_1$-$C_4$alkyl; with the exception of those compounds in which R is the radical S-$R_1$ and $R_2$ is $C_1$-$C_6$alkoxy or $C_1$-$C_6$alkylthio, by reacting: a benzothiadiazole compound of the formula II

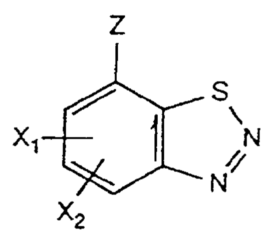

(II)

with an amino compound of the formula III

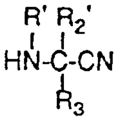

(III)

in the presence of a base and if appropriate with a catalyst, in inert solvents at temperatures of from -10° to 160°C, where Z is the radicals COOH, Hal-CO, $COOC_1$-$C_5$alkyl,

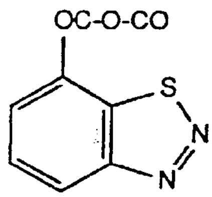

or

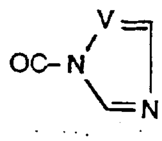

V is CH or N and Hal is halogen and R′ has the meaning of R as in formula I with the exception of S-$R_1$, and $R_2'$ has the meaning of $R_2$ as in formula I, with the exception of $C_1$-$C_6$alkoxy or $C_1$-$C_6$alkylthio, and $X_1$, $X_2$ and $R_3$ have the meanings indicated in the case of formula I.

**2.** A process for the preparation of compounds of the formula I in claim 1, in which the meaning S-$R_1$ is excluded for R (=R′) and in which $R_2$ is $C_1$-$C_6$alkoxy or $C_1$-$C_6$alkylthio (=$R_2''$), by:

a) reacting a benzothiadiazole compound of the formula II

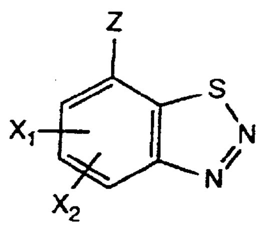

(II)

with an amino compound of the formula III′

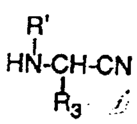

(III′)

in the presence of a base in inert solvents at temperatures of from -10° to 160°C to give an amide compound of the formula IV

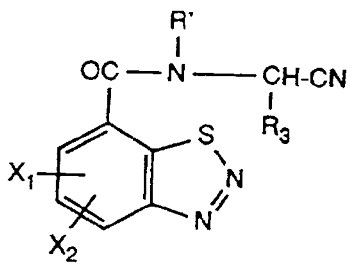

(IV)

and subsequently

b) converting the compound of the formula IV into a halogen compound of the formula V

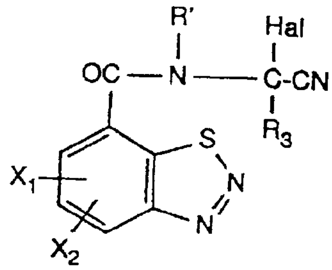

(V)

with a halogenating agent in inert solvents at temperatures of from -30° to 70°C and then

c) etherifying the compound of the formula V, after isolation or without isolation, with an alcohol or thiol of the formula VI

$HR_2''$ (VI)

in the presence of a base in inert solvents at temperatures of from -20° to 160°C, where Z is the radicals COOH, Hal-CO, $COOC_1$-$C_3$alkyl,

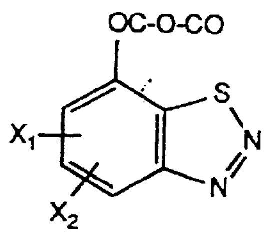

or

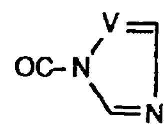

V is CH or N and Hal is halogen, and R′ has the meaning of R as in formula I with the exception of S-$R_1$, and $R_2''$ has the meaning of $C_1$-$C_6$alkoxy and $C_1$-$C_6$alkylthio, and $X_1$, $X_2$ and $R_3$ have the meanings indicated in the case of formula I.

3. Process for the preparation of compounds of the formula I in claim 1, in which R has the meaning of S-$R_1$, by reacting a compound of the formula Ia

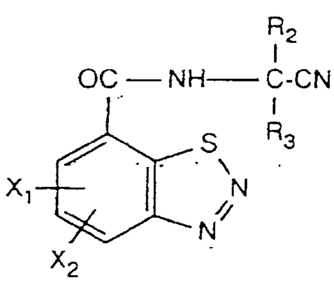

(Ia)

with a sulfenyl halide of the formula VII

Hal-S-$R_1$ (VII)

in the presence of a base in inert solvents at temperatures of from -10°C to 80°C, where Hal is halogen and $X_1$, $X_2$, $R_1$, $R_2$ and $R_3$ have the meanings indicated in the case of the formula I.

4. An agent for protecting plants against attack by microorganisms, which contains, besides customary carriers and auxiliary substances, at least one compound of claim 1 as the active component.

5. An agent according to claim 4, which contains at least one compound of claim 2 or 3 as the active component.

6. An agent according to claim 4, which contains as active component at least one compound of the formula I of claim 1 where: $X_1$ and $X_2$ independently of one another are hydrogen or fluorine; R is hydrogen, methyl, ethyl, allyl or the radical S-$R_1$; $R_1$ is trichloromethyl, dichlorofluoromethyl or 1,1,2,2-tetrachloroethyl.

7. An agent according to claim 4, which contains as active component at least one compound of the formula of claim 1 where: $X_1$ and $X_2$ independently of one another are hydrogen or fluorine; R is hydrogen; $R_2$ is $C_1$-$C_4$alkoxy, $C_1$-$C_4$alkylthio, or is furyl or thienyl, each of which is bonded in the 2- or 3-position, or is furyl or thienyl, each of which is substituted by 1 to 3 halogen and bonded in the 2- or 3-position; and $R_3$ is hydrogen or $C_1$-$C_3$alkyl.

8. An agent according to claim 4, which contains as active component at least one compound of the formula of claim 1 where: $X_1$ and $X_2$ independently of one another are hydrogen or fluorine; R is hydrogen, methyl, ethyl allyl or the radical S-$R_1$; $R_1$ is trichloromethyl, dichlorofluoromethyl or 1,1,2,2-tetrachloroethyl; $R_2$ is $C_1$-$C_4$alkyl, or $C_1$-$C_5$alkyl which is interrupted by oxygen, or $C_1$-$C_6$alkoxy, trichloromethyl, trifluoromethyl, 1,1,1-trifluoroethoxy, $C_1$-$C_6$alkylthio, or is furyl or thienyl, each of which is bonded in the 2- or 3-position, or is furyl or thienyl, each of which is substituted by 1 to 3 halogen and bonded in the 2- or 3-position; and $R_3$ is hydrogen or methyl.

9. An agent according to claim 4, which contains as active component at least one compound of the formula I of claim 1 where: $X_1$ and $X_2$ independently of one another are hydrogen or fluorine; R is hydrogen, methyl, ethyl allyl or the radical S-$R_1$; $R_1$ is trichloromethyl, dichlorofluoromethyl or 1,1,2,2-tetrachloroethyl; $R_2$ is $C_1$-$C_4$alkyl, or $C_1$-$C_5$alkyl which is interrupted by oxygen, or $C_1$-$C_6$alkoxy, trichloromethyl, trifluoromethyl, 1,1,1-trifluoroethoxy, $C_1$-$C_6$alkylthio, or is furyl or thienyl, each of which is bonded in the 2- or 3-position, or is furyl or thienyl, each of which is substituted by 1 to 3 halogen and bondeed in the 2- or 3- position; and $R_3$ is hydrogen.

10. An agent according to claim 4, which contains as active component at least one compound of the formula I of claim 1 where: $X_1$ and $X_2$ independently of one another are hydrogen or fluorine; R is hydrogen; $R_2$ is $C_1$-$C_6$alkoxy, $C_1$-$C_6$alkylthio, or is furyl or thienyl, each of which is bonded in the 2- or 3-position; and $R_3$ is hydrogen.

11. An agent according to claim 4, which contains as active component at least one compound from the group comprising:
N-(benzo-1,2,3-thiadiazole-7-carbonyl)-2-ethoxy-2-aminoacetonitrile;
N-(benzo-1,2,3-thiadiazole-7-carbonyl)-2-methoxy-2-aminoacetonitrile;
N-(benzo-1,2,3-thiadiazole-7-carbonyl)-2-(fur-2′-yl)-2-aminoacetonitrile;
N-(benzo-1,2,3-thiadiazole-7-carbonyl)-2-(thiophen-2′-yl)-2-aminoacetonitrile;
N-(benzo-1,2,3-thiadiazole-7-carbonyl)-2-(thiophen-3′-yl)-2-aminoacetonitrile;
N-(5-fluorobenzo-1,2,3-thiadiazole-7-carbonyl)-2-(thiophen-3′-yl)-2-aminoacetonitrile;
N-(4-fluorobenzo-1,2,3-thiadiazole-7-carbonyl)-2-(thiophen-3′-yl)-2-aminoacetonitrile;
N-(benzo-1,2,3-thiadiazole-7-carbonyl)-2-(fur-3′-yl)-2-aminoacetonitrile;
N-(benzo-1,2,3-thiadiazole-7-carbonyl)-2-methylthio-2-aminoacetonitrile;
N-(benzo-1,2,3-thiadiazole-7-carbonyl)-2-isopropylthio-2-aminoacetonitrile.

## Revendications

### Revendications pour les Etats contractants suivants : AT, BE, CH, DE, DK, FR, GB, GR, IT, LI, LU, NL, SE

1. Composés de formule I

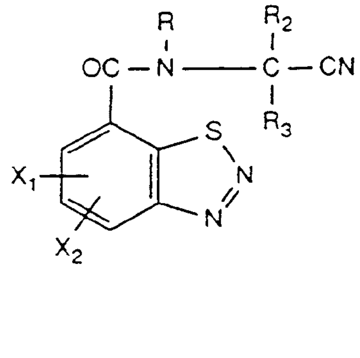

(I)

dans laquelle

| | |
|---|---|
| $X_1$ et $X_2$ | représentent chacun, indépendamment l'un de l'autre, l'hydrogène ou un halogène; |
| R | représente l'hydrogène, un groupe alkyle en C 1-C 4, alcényle en C 3-C 5 ou $SR_1$; |
| $R_1$ | représente un groupe alkyle en C 1-C 3 substitué par 3 à 6 atomes de fluor ou de chlore; |
| $R_2$ | représente un groupe alkyle en C 1-C 4, un groupe alkyle en C 1-C 5 interrompu par l'oxygène, un groupe alcoxy en C 1-C 6; un groupe alkyle en C 1-C 3 ou alcoxy en C 2-C 3 substitué par 1 à 3 atomes d'halogènes, un groupe alkylthio en C 1-C 6, un groupe furyle ou thiényle relié en position 2 ou 3, un groupe furyle ou thiényle substitué par 1 à 3 atomes d'halogènes et relié en position 2 ou 3; et |
| $R_3$ | représente l'hydrogène ou un groupe alkyle en C 1-C 4. |

2. Composés de formule I dans laquelle :

| | |
|---|---|
| $X_1$ et $X_2$ | représentent chacun, indépendamment l'un de l'autre, l'hydrogène, ou le fluor; |
| R | représente l'hydrogène, un groupe méthyle, éthyle. allyle ou $S-R_1$; |
| $R_1$ | représente un groupe trichlorométhyle, dichlorofluorométhyle ou 1,1,2,2-tétrachloréthyle. |

3. Composés de formule I dans laquelle :

| | |
|---|---|
| $X_1$ et $X_2$ | représentent chacun, indépendamment l'un de l'autre, l'hydrogène ou le fluor; |
| R | représente l'hydrogène; |
| $R_2$ | représente un groupe alcoxy en C 1-C 4, alkylthio en C 1-C 4, furyle ou thiényle relié en position 2 ou 3, furyle ou thiényle substitué par 1 à 3 atomes d'halogènes et relié en position 2 ou 3; et |
| $R_3$ | représente l'hydrogène ou un groupe alkyle en C 1-C 3. |

4. Composés de formule I selon revendication 1, dans laquelle :

| | |
|---|---|
| $X_1$ et $X_2$ | représentent chacun, indépendamment l'un de l'autre, l'hydrogène ou le fluor, R représente l'hydrogène, un groupe méthyle, éthyle, allyle ou le groupe $S-R_1$; |
| $R_1$ | présente un groupe trichlorométhyle, dichlorofluorométhyle ou 1,1,2,2-trichloréthyle; |
| $R_2$ | présente un groupe alkyle en C 1-C 4, un groupe alkyle en C 1-C 5 interrompu par l'oxygène, un groupe alcoxy en C 1-C 6, trichlorométhyle, trifluorométhyle, 1,1,1-trifluoréthoxy, alkylthio en C 1-C 6, furyle ou thiényle relié en position 2 ou 3, furyle ou thiényle substitué par 1 à 3 atomes d'halogènes et relié en position 2 ou 3; et |
| $R_3$ | représente l'hydrogène ou un groupe méthyle. |

5. Composés de formule I selon revendication 1, dans laquelle :

| | |
|---|---|
| $X_1$ et $X_2$ | représentent chacun, indépendamment l'un de l'autre, l'hydrogène ou le fluor; |
| R | représente l'hydrogène, un groupe méthyle, éthyle, allyle ou $S-R_1$; |
| $R_1$ | représente un groupe trichlorométhyle, dichlorofluorométhyle ou 1,1,2,2-trichloréthyle; |
| $R_2$ | représente un groupe alkyle en C 1-C 4, un groupe alkyle en C 1-C 5 interrompu par l'oxygène, un groupe alcoxy en C 1-C 6, trichlorométhyle, trifluorométhyle, 1,1,1-trifluoréthoxy, aîkylthio en C 1-C 6, furyle ou thiényle relié en position 2 ou 3, furyle ou thiényle substitué par 1 à 3 atomes d'halogènes et relié en position 2 ou 3; et |
| $R_3$ | représente l'hydrogène. |

6. Composés de formule I selon revendication 1, dans laquelle :

| | |
|---|---|
| $X_1$ et $X_2$ | représentent chacun, indépendamment l'un de l'autre, l'hydrogène ou le fluor; |
| R | représente l'hydrogène; |
| $R_2$ | présente un groupe alcoxy en C 1-C 6, alkylthio en C 1-C 6, furyle ou thiényle relié en position 2 ou 3; et |
| $R_3$ | représente l'hydrogène. |

7. Un composé selon revendication 1 pris dans le groupe suivant :
N-(benzo-1,2,3-thiadiazole-7-carbonyl) -2-éthoxy-2-aminoacétonitrile;
N-(benzo-1,2,3-thiadiazole-7-carbonyl)-2-méthoxy-2-aminoacétonitrile;
N-(benzo-1,2,3-thiadiazole-7-carbonyl)-2-(furyl-2'-yl)-2-aminoacétonitrile;
N-(benzo-1,2,3-thiadiazole-7-carbonyl)-2-(thiophène-2'-yl)-2-aminoacétonitrile;
N-(benzo-1,2,3-thiadiazole-7-carbonyl)-2-(thiophène-3'-yl)-2-aminoacétonitrile;
N-(5-fluorobenzo-1,2,3-thiadiazole-7-carbonyl)-2-(thiophène-3'-yl)-2-aminoacétonitrile;

N-(4-fluorobenzo-1,2,3-thiadiazole-7-carbonyl)-2-(thiophène-3′-yl)-2-aminoacétonitrile;
N-(benzo-1,2,3-thiadiazole-7-carbonyl)-2-(furyl-3′-yl)-2-aminoacétonitrile;
N-(benzo-1,2,3-thiadiazole-7-carbonyl)-2-méthylthio-2-aminoacétonitrile;
N-(benzo-1,2,3-thiadiazole-7-carbonyl)-2-isopropylthio-2-aminoacétonitrile.

**8.** Procédé de préparation des composés de formule I de la revendication 1, à l'exception de ceux pour lesquels R représente le groupe S-$R_1$ et $R_2$ représente un groupe alcoxy en C 1-C 6 ou alkylthio en C 1-C 6, selon lequel on fait réagir : un dérivé du benzothiadiazole de formule II

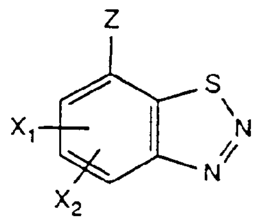

(II)

avec un dérivé aminé de formule III

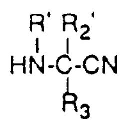

(III)

en présence d'une base et éventuellement d'un catalyseur dans un solvant inerte, à des températures de -10 à 160°C, Z représentant les groupes COOH, Hal-CO, COO-alkyle en C 1-C 5,

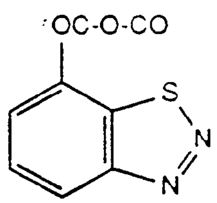

ou

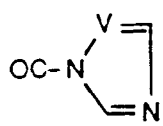

V représente CH ou N et Hal un halogène, R′ ayant les significations de R dans la formule I sauf le groupe S-$R_1$ et $R'_2$ les significations de $R_2$ dans la formule I sauf alcoxy en C 1-C 6 ou alkylthio en C 1-C 6, et $X_1$, $X_2$ et $R_3$ ayant les significations indiquées en référence à la formule I.

**9.** Procédé de préparation des composés de formule I de la revendication 1, pour lesquels R a une signification autre que S-$R_1$ (= R′) et $R_2$ représente un groupe alcoxy en C 1-C 6 ou alkylthio en C 1-C 6 (= $R''_2$) selon lequel

a) on fait réagir un dérivé du benzothiadiazole de formule II

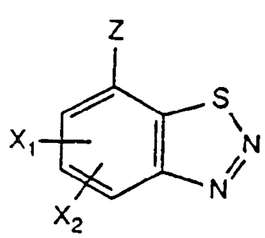

(II)

avec un dérivé aminé de formule III′

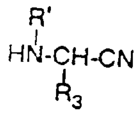

(III')

en présence d'une base dans un solvant inerte, à des températures de -10 à 160°C, la réaction donnant un amide de formule IV

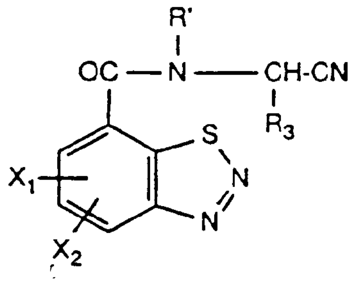

(IV)

b) qu'on convertit à l'aide d'un agent halogénant dans un solvant inerte, à des températures de - 30 à 70°C, en un dérivé halogéné de formule V

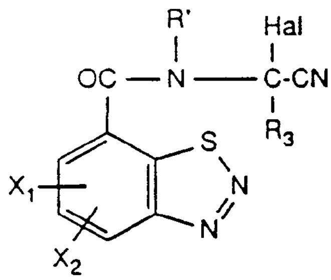

(V)

c) qu'on éthérifie après isolement ou sans l'isoler, à l'aide d'un alcool ou thiol de formule VI

$HR_2''$ (VI)

en présence d'une base, dans un solvant inerte, à des températures de -20 à 160°C, Z représentant les groupes COOH, Hal-CO ou COO-alkyle en C 1-C 3,

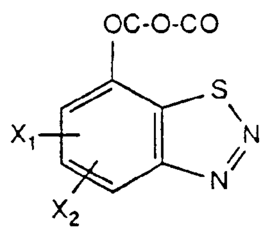

ou

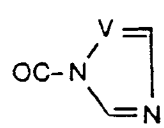

V représentant CH ou N et Hal un halogène, R' ayant les significations de R dans la formule I sauf le groupe $S-R_1$ et $R_2''$ représente un groupe alcoxy en C 1-C 6 ou alkylthio en C 1-C 6, $X_1$, $X_2$ et $R_3$ ayant les significations indiquées en référence à la formule I.

**10.** Procédé de préparation des composés de formule I de la revendication 1, pour lesquels R représente un groupe $S-R_1$, selon lequel on fait réagir un composé de formule Ia

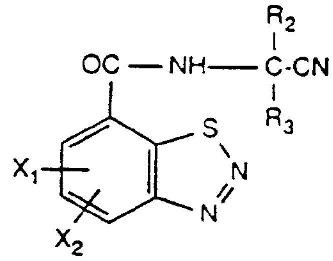

(Ia)

avec un halogénure de sulfényle de formule VII

$$\text{Hal-S-R}_1 \quad \text{(VII)}$$

en présence d'une base, dans un solvant inerte, à des températures de -10 à 80°C, Hal représentant un halogène et $X_1$, $X_2$, $R_1$, $R_2$ et $R_3$ ayant les significations indiquées en référence à la formule I.

**11.** Produit pour protéger les végétaux contre les attaques par des microorganismes, caractérisé en ce qu'il contient, avec des véhicules et produits auxiliaires usuels, au moins un composant actif consistant en un composé selon revendication 1.

**12.** Produit selon revendication 11, caractérisé en ce qu'il contient au moins un composant actif consistant en un composé selon les revendications 2 à 6.

**13.** Produit selon revendication 11, caractérisé en ce qu'il contient en tant que composant actif le composé de formule I de la revendication 7.

**14.** Procédé de préparation d'un produit agrochimique selon revendication 11, caractérisé en ce que l'on mélange intimement au moins un composé défini dans la revendication 1 avec des véhicules et produits auxiliaires solides ou liquides appropriés.

**15.** Utilisation des composés selon revendication 1 pour la protection des végétaux contre les attaques par des microorganismes phytopathogènes.

**16.** Utilisation de composés selon une des revendications 2 à 7 pour la protection des végétaux contre les attaques par des microorganismes phytopathogènes.

**17.** Procédé pour protéger les végétaux contre les attaques par des microorganismes phytopathogènes, caractérisé en ce que l'on applique sur la plante ou son habitat une substance active consistant en un composé selon revendication 1.

**18.** Procédé pour protéger les végétaux contre les attaques par des microorganismes phytopathogènes, caractérisé en ce que l'on applique sur la plante ou son habitat une substance active consistant en un composé selon l'une des revendications 2 à 7.

**19.** Procédé selon les revendications 17 et 18, caractérisé en ce que les microorganismes phytopathogènes sont des mycètes.

**Revendications pour l'Etat contractant suivant : ES**

**1.** Procédé de préparation des composés de formule I

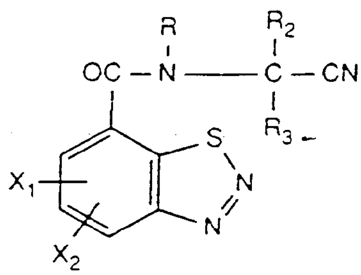

(I)

dans laquelle

$X_1$ et $X_2$ représentent chacun, indépendamment l'un de l'autre, l'hydrogène ou un halogène;

R représente l'hydrogène, un groupe alkyle en C 1-C 4, alcényle en C 3-C 5 ou $S\text{-}R_1$;

$R_1$ présente un groupe alkyle en C 1-C 3 substitué par 3 à 6 atomes de fluor ou de chlore;

$R_2$ présente un groupe alkyle en C 1-C 4, un groupe alkyle en C 1-C 5 interrompu par l'oxygène, un groupe alcoxy en C 1-C 6, un groupe alkyle en C 1-C 3 ou alcoxy en C 2-C 3 substitué par 1 à 3 atomes d'halogènes, un groupe alkylthio en C 1-C 6, un groupe furyle ou thiényle relié en position 2 ou 3, un groupe furyle ou thiényle substitué par 1 à 3 atomes d'halogènes et relié en position 2 ou 3; et

$R_3$ présente l'hydrogène ou un groupe alkyle en C 1-C 4;

à l'exception des composés pour lesquels R représente le groupe $S\text{-}R_1$ et $R_2$ représente un groupe alcoxy en C 1-C 6 ou alkylthio en C 1-C 6, selon lequel on fait réagir un dérivé du benzothiadiazole de formule II

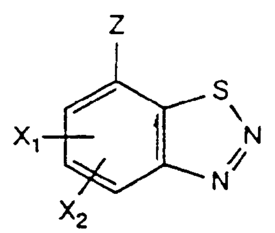

(II)

avec un dérivé aminé de formule III

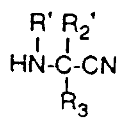

(III)

en présence d'une base et éventuellement d'un catalyseur dans un solvant inerte à des températures de -10 à 160°C, Z représentant les groupes COOH, Hal-COO ou COO-alkyle en C 1-C 5,

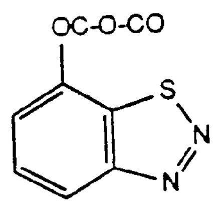

ou

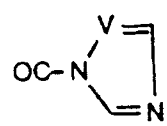

V représente CH ou N et Hal un halogène et R' a les significations de R dans la formule I à l'exception de $S\text{-}R_1$ et $R'_2$ les significations de $R_2$ dans la formule I à l'exception d'un groupe alcoxy en C 1-C 6 ou alkylthio en C 1-C 6, $X_1$, $X_2$ et $R_3$ ayant les significations indiquées en référence à la formule I.

**2.** Procédé de préparation des composés de formule I de la revendication 1, pour lesquels R a une signification autre que $S\text{-}R_1$ (= R') et $R_2$ représente un groupe alcoxy en C 1-C 6 ou alkylthio en C 1-C 6 (= $R''_2$) selon lequel

a) on fait réagir un dérivé du benzothiadiazole de formule II

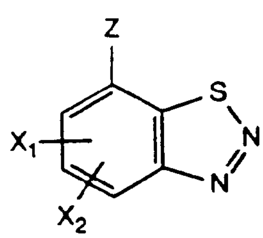

(II)

avec un dérivé aminé de formule III′

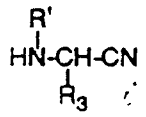

(III')

en présence d'une base, dans un solvant, inerte, à des températures de -10 à 160°C, la réaction donnant un amide de formule IV

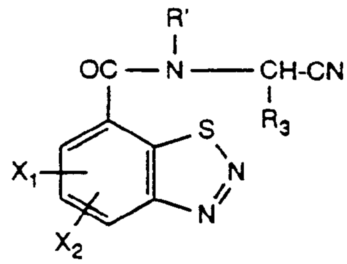

(IV)

b) qu'on convertit à l'aide d'un agent halogénant dans un solvant inerte, à des températures de -30 à 70°C, en un dérivé halogéné de formule V

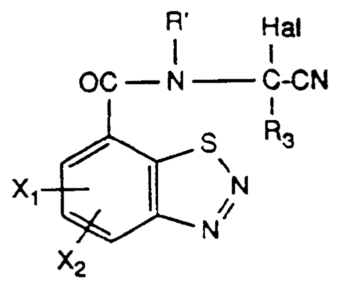

(V)

c) qu'on éthérifie après isolement ou sans l'isoler à l'aide d'un alcool ou d'un thiol de formule VI

$HR_2''$ (VI)

en présence d'une base, dans un solvant inerte, à des températures de -20 à 160°C, Z représentant les groupes COOH, Hal-CO, COO-alkyle en C 1-C 3

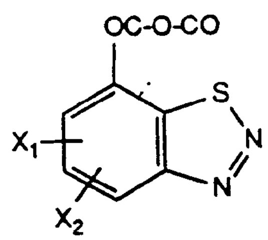

ou

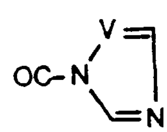

V représente CH ou N et Hal un halogène, R′ ayant les significations de R dans la formule I sauf S-$R_1$, et $R_2''$ représentant un groupe alcoxy en C 1-C 6 ou alkylthio en C 1-C 6, $X_1$, $X_2$ et $R_3$ ayant les significations indiquées en référence à la formule I.

**3.** Procédé de préparation des composés de la formule I de la revendication 1, pour lesquels R représente un groupe S-$R_1$, selon lequel on fait réagir un composé de formule Ia

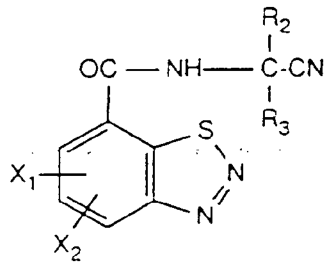

(Ia)

avec un halogénure de sulfényle de formule VII

Hal-S-$R_1$ (VII)

en présence d'une base, dans un solvant inerte, à des températures de -10 à 80°C, Hal représentant un halogène et $X_1$, $X_2$, $R_1$, $R_2$ et $R_3$ ayant les significations indiquées en référence à la formule I.

**4.** Produit pour protéger les végétaux par les attaques par les microorganismes, caractérisé en ce qu'il contient, avec des véhicules et produits auxiliaires usuels, au moins un composant actif consistant en un composé selon revendication 1.

**5.** Produit selon revendication 4, caractérisé en ce qu'il contient au moins un composant actif consistant en un composé selon revendication 2 ou 3.

**6.** Produit selon revendication 4, caractérisé en ce qu'il contient au moins un composant actif consistant en un composé de formule I de la revendication 1 dans laquelle :

$X_1$ et $X_2$ représentent chacun, indépendamment l'un de l'autre, l'hydrogène ou le fluor;

R représente l'hydrogène, un groupe méthyle, éthyle, allyle ou le groupe S-$R_1$;

$R_1$ représente un groupe trichlorométhyle, dichlorofluorométhyle ou 1,1,2,2-tétrachloréthyle.

**7.** Produit selon revendication 4, caractérisé en ce qu'il contient au moins un composant actif consistant en un composé de formule I dans laquelle :

$X_1$ et $X_2$ représentent chacun, indépendamment l'un de l'autre, l'hydrogène ou le fluor;

R représente l'hydrogène;

$R_2$ représente un groupe alcoxy en C 1-C 4, alkylthio en C 1-C 4, furyle ou thiényle relié en position 2 ou 3, furyle ou thiényle substitué par 1 à 3 atomes d'halogènes et relié en position 2 ou 3; et

$R_3$ représente l'hydrogène ou un groupe alkyle en C 1-C 3.

**8.** Produit selon revendication 4, caractérisé en ce qu'il contient au moins un composant actif consistant en un composé de formule I de la revendication 1 dans laquelle :

$X_1$ et $X_2$ représentent chacun, indépendamment l'un de l'autre, l'hydrogène ou le fluor, R représente l'hydrogène, un groupe méthyle, éthyle, allyle ou le groupe S-$R_2$;

$R_1$ représente un groupe trichlorométhyle, dichlorofluorométhyle ou 1,1,2,2-tétrachloréthyle,

$R_2$ représente un groupe alkyle en C 1-C 4, un groupe alkyle en C 1-C 5 interrompu par l'oxygène, un groupe alcoxy en C 1-C 6, trichlorométhyle, trifluorométhyle, 1,1,1-trifluoréthoxy, alkylthio en C 1-C 6, furyle ou thiényle relié en position 2 ou 3, furyle ou thiényle substitué par 1 à 3 atomes d'halogènes et relié en position 2 ou 3; et

$R_3$ représente l'hydrogène ou un groupe méthyle.

**9.** Produit selon revendication 4, caractérisé en ce qu'il contient au moins un composant actif consistant en un composé de formule I de la revendication 1 dans laquelle :

$X_1$ et $X_2$ représentent chacun, indépendamment l'un de l'autre, l'hydrogène ou le fluor;

R représente l'hydrogène, un groupe méthyle, éthyle, allyle ou S-$R_1$;

$R_1$ représente un groupe trichlorométhyle, dichlorofluorométhyle ou 1,1,2,2-tétrachloréthyle;

$R_2$ représente un groupe alkyle en C 1-C 4, un groupe alkyle en C 1-C 5 interrompu par l'oxygène, un groupe alcoxy en C 1-C 6, trichlorométhyle, trifluorométhyle, 1,1,1-trifluoréthoxy, alkylthio en C 1-C 6, furyle ou thiényle relié en position 2 ou 3, furyle ou thiényle substitué par 1 à 3 atomes d'halogènes et relié en position 2 ou 3; et

$R_3$ représente l'hydrogène.

**10.** Produit selon revendication 4, caractérisé en ce qu'il contient au moins un composant actif consistant en un composé de formule I de la revendication 1 dans laquelle :

$X_1$ et $X_2$ représentent chacun, indépendamment l'un de l'autre, l'hydrogène ou le fluor;

R représente l'hydrogène;

$R_2$ représente un groupe alcoxy en C 1-C 6, alkylthio en C 1-C 6, ou furyle ou thiényle relié en position 2 ou 3; et

$R_3$ représente l'hydrogène.

**11.** Produit selon revendication 4, caractérisé en ce qu'il contient au moins un composant actif consistant en un composé du groupe suivant :

N-(benzo-1,2,3-thiadiazole-7-carbonyl)-2-éthoxy-2-aminoacétonitrile;
N-(benzo-1,2,3-thiadiazole-7-carbonyl)-2-méthoxy-2-aminoacétonitrile;
N-(benzo-1,2,3-thiadiazole-7-carbonyl)-2-(furyl-2′-yl)-2-aminoacétonitrile;
N-(benzo-1,2,3-thiadiazole-7-carbonyl)-2-(thiophène-2′-yl)-2-aminoacétonitrile;
N-(benzo-1,2,3-thiadiazole-7-carbonyl)-2-(thiophène-3′-yl)-2-aminoacétonitrile;
N-(5-fluorobenzo-1,2,3-thiadiazole-7-carbonyl)-2-(thiophène-3′-yl)-2-aminoacétonitrile;
N-(4-fluorobenzo-1,2,3-thiadiazole-7-carbonyl)-2-(thiophène-3′-yl)-2-aminoacétonitrile;
N-(benzo-1,2,3-thiadiazole-7-carbonyl)-2-(furyl-3′-yl)-2-aminoacétonitrile;
N-(benzo-1,2,3-thiadiazole-7-carbonyl)-2-méthylthio-2-aminoacétonitrile;
N-(benzo-1,2,3-thiadiazole-7-carbonyl)-2-isopropylthio-2-aminoacétonitrile.